# EUROPEAN PATENT APPLICATION

(11) **EP 4 079 310 A1**
(43) Date of publication of application: **26.10.2022**
(21) Application number: 21169361.9
(22) Date of filing: 20.04.2021
(51) Int. Cl.: A61K 31/706, A61P 25/16, A61P 25/28, A61K 31/7084, A61P 25/14

(54) **NMN AND DERIVATIVES FOR ITS USE IN THE TREATMENT OF ALPHA-SYNUCLEINOPATHIES**

(71) Applicant: Nuvamid SA, 1003 Lausanne (CH)
(72) Inventor: BERMOND, Guillaume, 13007 MARSEILLE (FR); GARCON, Laurent, 13960 SAUSSET LES PINS (FR)
(74) Representative: August Debouzy

(57) **Abstract**

The invention relates to compounds of formula (I) and/or of formula (la) for the prevention and/or treatment of an alpha-synucleinopathy such as dementia with Lewy bodies (DLB) and multiple system atrophy (MSA), as well as compositions and kit of parts comprising the same.

## Description

### FIELD OF THE INVENTION

The invention also relates to compounds of formula (I) and/or (Ia) and at least one pharmaceutically acceptable excipient for use in the prevention and/or treatment of an alpha-synucleinopathy chosen amongst dementia with Lewy bodies (DLB) and multiple system atrophy (MSA), as well as compositions and kit of parts comprising the same.

### TECHNICAL BACKGROUND

Alpha-synucleinopathies are a group of orphan neurodegenerative diseases characterised by the abnormal accumulation of aggregates of alpha-synuclein protein in neurons, nerve fibres or glial cells. The synucleinopathies have shared features of parkinsonism, impaired cognition, sleep disorders, and visual hallucinations.

Dementia with Lewy bodies (DLB) is classified as G31.8 in the 10^{th} International Classification of Diseases (ICD 10) as published in the Official Bulletin No 2020/9 bis(https://www.atih.sante.fr/sites/default/files/public/content/3706/cim-10 2020 bo.pdf). This disease is characterized by changes in thinking, movement, behaviour, and mood. Dementia with Lewy bodies and Parkinson's disease (PD) dementia have similar neuropathological features, but these features are highly variable, and the conditions cannot be distinguished on pathological features alone. Generally, dementia with Lewy bodies is distinguished from PD dementia by the time frame in which dementia symptoms appear relative to parkinsonian symptoms and is diagnosed when cognitive symptoms begin before or at the same time as parkinsonism. PD dementia is the diagnosis when PD is already well established before the dementia occurs. Between 5% and 25% of diagnosed dementias in older adults are due to Lewy body dementias (DLB). DLB usually develops after the age of 50. Men are more likely to be diagnosed than women. There is currently no treatment for these patients. Administration of L-dopamine does not alleviate their symptoms.

Multisystemic Atrophy (MSA) is a rapidly progressing neurodegenerative disease with a sporadic adult prevalence of between 3.4 and 4.9 per 100,000 people. MSA is classified as atypical parkinsonism with symptoms including bradykinesia, rigidity and postural instability (postural hypotension). Other non-motor symptoms include urogenital, gastrointestinal and cardiovascular insufficiency. Sleep, cognitive impairment, respiratory problems and behavioural symptoms also occur during the progression of the disease. MSA is classified in the category G23 in ICD 10. MSA is defined in two subtypes, the Parkinsonian subtype (G23.2 in ICD 10) with patients with Parkinsonian-type symptoms, and the cerebellar subtype (G23.3 in ICD 10) for which patients suffer from cerebellar ataxia. MSA is characterized in part by abnormal accumulation of alpha-synuclein in oligodendrocytes, inflammation, demyelination and synaptic and neuronal loss. Infiltration of immune cells into the brain tissue leads to significant inflammation. Extracellular alpha-synuclein plays an essential role in the activation of microglia, which leads to the secretion of inflammatory cytokines and thus to neurodegeneration.

Because some symptoms of MSA and DLB are similar to those of Parkinson disease and because accumulation of abnormal aggregates of alpha-synuclein protein has also been observed in Parkinson disease, administration of levodopa (L-dopamine), with or without carbidopa, which is an effective treatment of Parkinson disease, has been tried with patients diagnosed with MSA or DLB. However, contrarily to PD patients who respond well to levodopa, only 1.5% of MSA patients respond to this treatment. In addition, the response is transient: at first levodopamine is very effective in MSA patients but becomes ineffective after a year or two, contrarily to PD patients who first respond slowly to levodopa but who then notice a significant improvement with the treatment. Patients with DLB are not improved by the administration of L-dopamine.

Other treatments of MSA and DLB include physiotherapy, speech therapy as well as conventional treatments for incontinence, constipation, heart disease and erectile dysfunction.

There is therefore a need to develop new treatments for alpha-synucleinopathies such as dementia with Lewy bodies (DLB) and multiple system atrophy (MSA), preferably MSA. There is also a need to develop such treatments which are well tolerated by patients, do not worsen any of their other symptoms, and are compatible with a chronic administration.

### SUMMARY OF THE INVENTION

These goals are achieved, at least in part, by the present invention.

The present invention relates to a compound of formula (I): 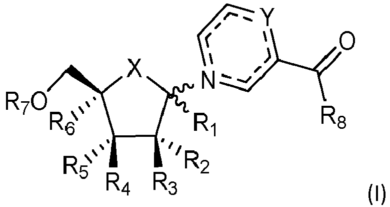 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 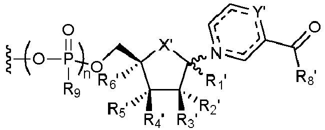 wherein n is an integer chosen amongst 1, 2 or 3; wherein:
   R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
   R11 is selected from C1-C10 alkyl, C3-C10 cycloalkyl, C5-C18 aryl, C1-C10 alkylaryl, substituted C5-C12 aryl, C1-C10 heteroalkyl, C3₋C10 heterocycloalkyle, C1-C10 haloalkyl, heteroaryl, -(CH2)nC(O)(C1-C15)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of R11a is independently selected from H and (C1-C6) alkyl and R11b is independently selected from OH, C1-C6 alkoxy, NH2, NH(C1-C6 alkyl) or N(C1-C6 alkyl)2 ;
   R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
   R13 and R14 are independently selected from H, C1-C8 alkyl and C1-C8 alkyl aryl;
   Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
   R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
   R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
   R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl;
   Y is selected from CH, CH2, C(CH3)2 and CCH3;
   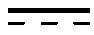 represents a single or double bond according to Y; and
   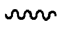 represents the alpha or beta anomer depending on the position of R1,
or a compound of formula (Ia) 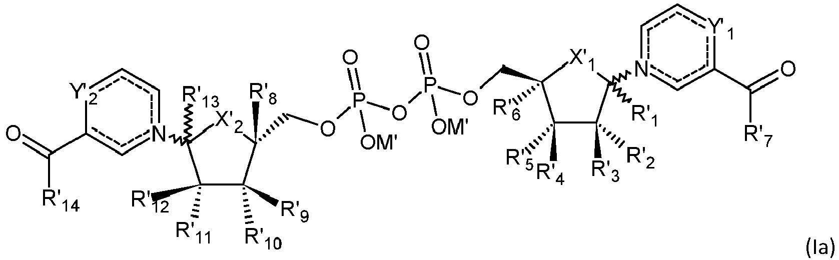 or pharmaceutically acceptable salts, hydrates, crystals, stereoisomers and/or solvates thereof, wherein:
X'1 and X'2 are independently selected from O, CH2, S, Se, CHF, CF2 and C=CH2;
R'1 and R'13 are independently selected from H, azido, cyano, C1-C8 alkyl, C1-C8 thio-alkyl, C1-C8 heteroalkyl and OR; wherein R is selected from H and C1-C8 alkyl;
R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid ;
R'6 and R'8 are independently selected from H, azido, cyano, C1-C8 alkyl and OR; wherein R is selected from H and C1-C8 alkyl;
R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
Y'1 and Y'2 are independently selected from CH, CH2, C(CH3)2 or CCH3;
M' is selected from H or a suitable counterion;
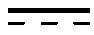 represents a single or a double bound depending on Y'1 and Y'2; and
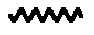 represents the alpha or beta anomer depending on the position of R'1 and R'13,
and their combinations for their use in the prevention and/or treatment of an alpha-synucleinopathy.

Advantageously, the compound according to the invention is selected from compounds I-A to I-J from Table 1 below ci-dessous or a pharmaceutically acceptable salt or solvate thereof:

**[Table 1]**

| **Composés (anomères)** | **Structure** |
|---|---|
| I-A (beta) | 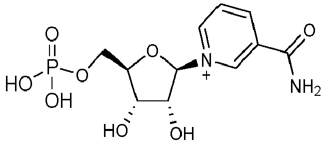 |
| I-B (alpha) | 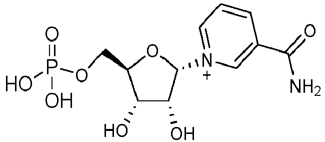 |
| I-C (beta) | 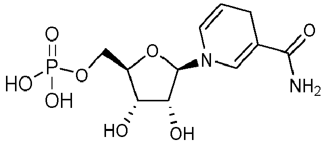 |
| I-D (alpha) | 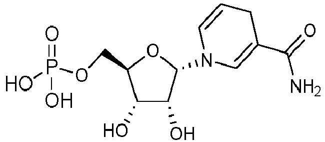 |
| I-E (beta) | 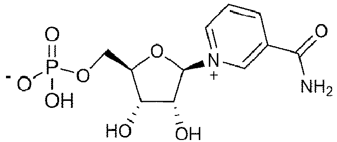 |
| I-F (alpha) | 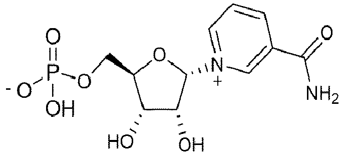 |
| I-G (beta) | 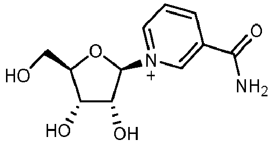 |
| I-H (alpha) | 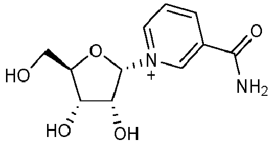 |
| I-I (beta) | 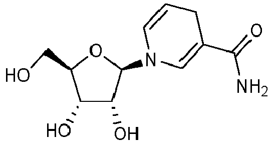 |
| I-J (alpha) | 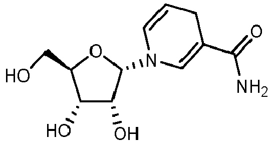 |

Advantageously, preferred compound of the invention are compounds I-A to I-F or a pharmaceutically acceptable salt or solvate thereof, more preferably IB, ID or IF.

Advantageously, the compound of formula (I) is dihydro-nicotinamide mononucleotide (NMN-H) of the following formula:

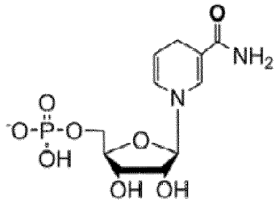

Advantageously, preferred compounds of the invention are compounds la-A to Ia-I, listed in table 2:

**[Table 2]**

| Compound | Structure |
|---|---|
| Ia-A (beta, beta) | 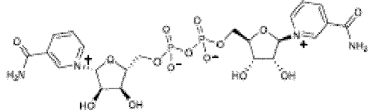 |
| Ia-B (beta, alpha) | 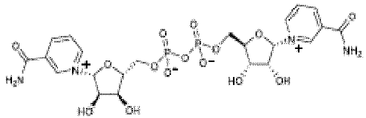 |
| Ia-C (alpha, alpha) | 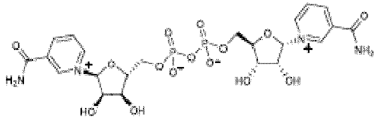 |
| Ia-D (beta, beta) | 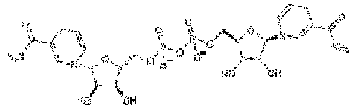 |
| Ia-E (beta, alpha) | 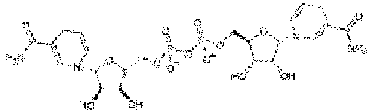 |
| Ia-F (alpha, alpha) | 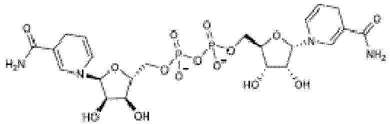 |
| Ia-G (beta, beta) | 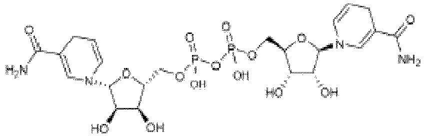 |
| Ia-H (beta, alpha) | 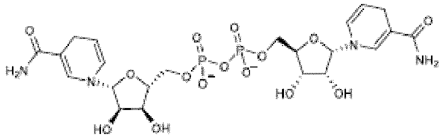 |
| Ia-I (alpha, alpha) | 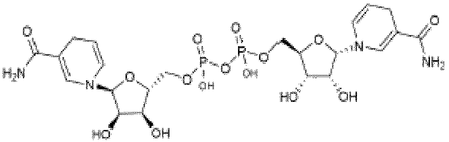 |

Advantageously, preferred compound of the invention is compound of formula la-C or la-F or Ia-I.

Advantageously, preferred compound of the invention is compound of formula la-B or la-E or la-H.

Advantageously, the alpha-synucleinopathy is preferably MSA.

Preferably, the MSA is chosen amongst MSA-predominant Parkinsonism (MSA-P) or the MSA-predominant cerebellar ataxia (MSA-C).

Preferably, the alpha-synucleinopathy is not Parkinson disease.

Advantageously, compound of formula (I) as described herein and/or compounds of formula (Ia) as described herein are for use in the treatment of an alpha-synucleinopathy.

Advantageously, compound of formula I and/or compound of formula Ia may be used in an amount between 0.01 mg/kg/day and 1000 mg/kg/day, preferably between 1 mg/kg/day and 100 mg/kg/day, more preferably between 5 mg/kg/day and 50 mg/kg/day, even more preferably between 10 and 20 mg/kg/day.

Advantageously, compound of formula I and/or compound of formula Ia may be administered orally, ocularly, sublingually, parenterally, transcutaneously, vaginally, epidurally, intravesically, rectally or by inhalation.

Preferably, compound of formula I and/or compound of formula Ia may be administered orally or parenterally.

Advantageously, compound of formula I and/or compound of formula Ia may be used in mammals, preferably humans.

Advantageously, compound of formula I and/or compound of formula Ia may be used in combination with at least one further therapeutic ingredient.

Advantageously, the at least one further therapeutic ingredient is chosen amongst a dopaminergic precursor, a monoamine oxidase type B inhibitor, an anticholinergic agent, a dopaminergic agonist, a noradrenaline precursor, a muscarinic antagonist, an alpha-blocker, a beta-adrenergic agonist, a corticoid, a phosphodiesterase inhibitor, a vasopressin V2 receptor agonist and their combinations.

Preferably, the at least one further therapeutic ingredient may be chosen amongst levodopa, carbidopa, oxybutine chloride, tolterodine, tamsulosine, mirabegron, tadalafil, sildenafil, a laxative, an anticholinergic agent, an acetylcholinesterase (ACE) inhibitor, a benzodiazepine, a dopaminergic agent, zolpidem, modafinil, armodafinil their combinations.

More preferably, the further therapeutic ingredient is levodopa (L-dopamine).

The present invention also relates to a composition comprising at least one compound of formula I and/or compound of formula la, one of its pharmaceutically acceptable salts and at least one pharmaceutically acceptable excipient for use in the prevention and/or treatment of an alpha-synucleinopathy chosen amongst dementia with Lewy bodies (DLB) and multiple system atrophy (MSA).

Advantageously, the alpha-synucleinopathy is preferably MSA.

Preferably, the alpha-synucleinopathy is not Parkinson disease.

Advantageously, the composition according to the invention is in the form of a tablet, a capsule, a sachet, a granule, a soft capsule, a lozenge, a lyophilisate, a suspension, a gel, a syrup, a solution, a water/oil emulsion, an oil/water emulsion, oil, cream, milk, spray, ointment, ampoule, suppository, eye drops, vaginal egg, vaginal capsule, liquid for inhalation, dry powder inhaler, pressurised metered dose inhaler.

Preferably, the composition according to the invention is in the form of a gastro-resistant capsule or a sublingual tablet.

Advantageously, the composition according to the invention is a pharmaceutical composition.

Advantageously, the composition according to the invention is a dietary supplement.

The present invention also relates to a composition comprising compound of formula I and/or compound of formula la, one of their pharmaceutically acceptable salts, at least one pharmaceutically acceptable excipient and at least one further therapeutic ingredient for its use according to the invention.

Advantageously, the at least one further therapeutic ingredient is as described herein.

The present invention also relates to a kit of parts comprising at least compound of formula I and/or compound of formula la, one of their pharmaceutically acceptable salts, and at least one further part.

Advantageously, the at least one further part may be an information leaflet.

Advantageously, the at least one further part may be at least one further therapeutic ingredient as described herein.

Preferably, the at least one further part comprises an information leaflet and at least one further therapeutic ingredient as described herein.

Advantageously, the kit of parts can be used in the treatment and/or prevention of an alpha-synucleinopathy chosen amongst dementia with Lewy bodies (DLB) and multiple system atrophy (MSA) as described herein.

Preferably, the kit of parts can be used in the treatment and/or prevention of an alpha-synucleinopathy chosen amongst dementia with Lewy bodies (DLB) and multiple system atrophy (MSA) as described herein.

This invention also relates to the use of a compound as described hereinabove in the manufacture of a medicament for the treatment and/or prevention of an alpha-synucleinopathy chosen amongst dementia with Lewy bodies (DLB) and multiple system atrophy (MSA), as described herein.

### DEFINITIONS

The definitions and explanations below are for the terms as used throughout the entire application, including both the specification and the claims.

When describing the compounds of the invention, the terms used are to be construed in accordance with the following definitions, unless indicated otherwise.

Unless indicated otherwise, the nomenclature of substituents that are not explicitly defined herein are arrived at by naming the adjacent functionality toward the point of attachment followed by the terminal portion of the functionality. For example, the substituent "arylalkyl" refers to the group -(aryl)-(alkyl).

In the present invention, the following terms have the following meanings.

The term "alkyl" by itself or as part of another substituent refers to a hydrocarbyl radical of Formula CnH2n+1 wherein n is a number greater than or equal to 1. Generally, alkyl groups of this invention comprise from 1 to 12 carbon atoms, preferably from 1 to 8 carbon atoms, more preferably from 1 to 6 carbon atoms, still more preferably 1 to 2 carbon atoms. Alkyl groups may be linear or branched and may be substituted as indicated herein. Suitable alkyl groups include methyl, ethyl, n-propyl, i-propyl, n- butyl, i-butyl, s-butyl and t-butyl, pentyl and its isomers (e.g. n-pentyl, iso-pentyl), and hexyl and its isomers (e.g. n-hexyl, iso-hexyl). Preferred alkyl groups include methyl, ethyl, n-propyl, i-propyl, n-butyl, i-butyl, s-butyl, t-butyl, n-pentyl, n-hexyl, n-heptyl, n-octyl, n-nonyl and n-decyl. Saturated branched alkyls include, without being limited to, isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl,
5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl.

Suitable alkyl groups include methyl, ethyl, n-propyl, i-propyl, n-butyl, i-butyl, s-butyl and t-butyl, pentyl and its isomers (e.g. n-pentyl, iso-pentyl), hexyl and its isomers (e.g. n-hexyl, isohexyl), heptyl and its isomers (e.g. heptyl-heptyl, iso-heptyl), octyl and its isomers (e.g. n-octyl, iso-octyl), nonyl and its isomers (e.g. n-nonyl, iso-nonyl), decyl and its isomers (e.g. n-decyl, iso-decyl), undecyl and its isomers, dodecyl and its isomers. Preferred alkyl groups are methyl, ethyl, n-propyl, i-propyl, n-butyl, i-butyl, s-butyl and t-butyl. Cx-Cy-alkyl refers to alkyl groups which comprise x to y carbon atoms, x and y being included.

When the suffix "ene" ("alkylene") is used in conjunction with an alkyl group, this is intended to mean the alkyl group as defined herein having two single bonds as points of attachment to other groups. The term "alkylene" includes methylene, ethylene, methylmethylene, propylene, ethylethylene, and 1,2-dimethylethylene.

The term "alkenyl" as used herein refers to an unsaturated hydrocarbyl group, which may be linear or branched, comprising one or more carbon-carbon double bonds. Suitable alkenyl groups comprise between 2 and 12 carbon atoms, preferably between 2 and 8 carbon atoms, still more preferably between 2 and 6 carbon atoms. Examples of alkenyl groups are ethenyl, 2- propenyl, 2-butenyl, 3-butenyl, 2-pentenyl and its isomers, 2-hexenyl and its isomers, 2,4-pentadienyl and the like.

The term "alkynyl" as used herein refers to a class of monovalent unsaturated hydrocarbyl groups, wherein the unsaturation arises from the presence of one or more carbon-carbon triple bonds. Alkynyl groups typically, and preferably, have the same number of carbon atoms as described above in relation to alkenyl groups. Non limiting examples of alkynyl groups are ethynyl, 2- propynyl, 2-butynyl, 3-butynyl, 2-pentynyl and its isomers, 2-hexynyl and its isomers-and the like.

The term "aryl" as used herein refers to a polyunsaturated, aromatic hydrocarbyl group having a single ring (i.e. phenyl) or multiple aromatic rings fused together (e.g. naphtyl) or linked covalently, typically containing 5 to 12 atoms; preferably 6 to 10, wherein at least one ring is aromatic. The aromatic ring may optionally include one to two additional rings (either cycloalkyl, heterocyclyl or heteroaryl) fused thereto. Aryl is also intended to include the partially hydrogenated derivatives of the carbocyclic systems enumerated herein. Non- limiting examples of aryl comprise phenyl, biphenylyl, biphenylenyl, 5- or 6-tetralinyl, naphthalen-1- or -2-yl, 4-, 5-, 6 or 7-indenyl, 1- 2-, 3-, 4- or 5- acenaphtylenyl, 3-, 4- or 5-acenaphtenyl, 1- or 2-pentalenyl, 4- or 5-indanyl, 5-, 6- , 7- or 8-tetrahydronaphthyl, 1,2,3,4-tetrahydronaphthyl, 1,4-dihydronaphthyl, 1-, 2-, 3-, 4- or 5-pyrenyl.

The term "cycloalkyl" as used herein is a cyclic alkyl group, that is to say, a monovalent, saturated, or unsaturated hydrocarbyl group having 1 or 2 cyclic structures. Cycloalkyl includes monocyclic or bicyclic hydrocarbyl groups. Cycloalkyl groups may comprise 3 or more carbon atoms in the ring and generally, according to this invention comprise from 3 to 10, more preferably from 3 to 8 carbon atoms still more preferably from 3 to 6 carbon atoms. Examples of cycloalkyl groups include but are not limited to cyclopropyl, cyclobutyl, cyclopentyl, cyclohexyl, with cyclopropyl being particularly preferred.

As used herein, the term "antibody" means a polypeptide comprising a framework region from an immunoglobulin gene or fragments thereof that specifically binds and recognizes an antigen, e.g., myostatin. Use of the term antibody is meant to include whole antibodies, including single-chain whole antibodies, and antigen-binding fragments thereof. The term "antibody" includes bispecific antibodies and multispecific antibodies so long as they exhibit the desired biological activity or function.

As used herein, the term "antibody-related polypeptide" means antigen-binding antibody fragments, including single-chain antibodies, that can comprise the variable region(s) alone, or in combination, with all or part of the following polypeptide elements: hinge region, CH1, CH2, and CH3 domains of an antibody molecule. Also included in the disclosure are any combinations of variable region(s) and hinge region, CH1, CH2, and CH3 domains. Antibody- molecules of the present technology include, e.g., but are not limited to, Fab, Fab' and F(ab')2, Fd, single-chain Fvs (scFv), single-chain antibodies, disulfide-linked Fvs ( sdFv) and fragments comprising either a V L or V H domain. Examples include: (i) a Fab fragment, a monovalent fragment consisting of the VL, VH, CL and CH1 domains; (ii) a F(ab')z fragment, a bivalent fragment comprising two Fab fragments linked by a disulphide bridge at the hinge region; (iii) a Fd fragment consisting of the VH and CH1 domains; (iv) a Fv fragment consisting of the VL and VH domains of a single arm of an antibody, (v) a dAb fragment (Ward, et al., Nature 341: 544-546, 1989), which consists of a V H domain; and (vi) an isolated complementarity determining region (CDR). As such "antibody fragments" can comprise a portion of a full-length antibody, generally the antigen binding or variable region thereof. Examples of antibody fragments include Fab, Fab', F(ab')2, and Fv fragments; diabodies; linear antibodies; single-chain antibody molecules; and multispecific antibodies formed from antibody fragments. Single-chain antibody molecules may comprise a polymer with a number of individual molecules, for example, dimmer, trimer or other polymers.

As used herein, the term "CDR-grafted antibody" means an antibody in which at least one CDR of an "acceptor" antibody is replaced by a CDR "graft" from a "donor" antibody possessing a desirable antigen specificity.

As used herein, the term "chimeric antibody" means an antibody in which the Fc constant region of a monoclonal antibody from one species (e.g., a mouse Fc constant region) is replaced, using recombinant DNA techniques, with an Fc constant region from an antibody of another species (e.g., a human Fc constant region). See generally Better, et al., Science 240: 1041-1043, 1988; Liu, et al., Proc Natl Acad Sci USA 84: 3439-3443, 1987; Liu, et al., J Immuno/139: 3521-3526, 1987; Sun, et al., Proc Natl Acad Sci USA 84: 214-218, 1987; Nishimura, et al., Cancer Res 47:999-1005, 1987; Wood, et al., Nature 314:446-449, 1985; and Shaw, et al., JNatl Cancer Inst 80: 1553-1559, 1988.

As used herein, the term "diabodies" refers to small antibody fragments with two antigen-binding sites, which fragments comprise a heavy-chain variable domain (VH) connected to a light-chain variable domain (VL) in the same polypeptide chain (VH VL). By using a linker that is too short to allow pairing between the two domains on the same chain, the domains are forced to pair with the complementary domains of another chain and create two antigen binding sites. Diabodies are described more fully in Hollinger, et al., Proc. Natl. Acad. Sci. USA, 90: 6444-6448 (1993).

As used herein, the term "gene" means a segment of DNA that contains all the information for the regulated biosynthesis of an RNA product, including promoters, exons, introns, and other untranslated regions that control expression.

As used herein, the term "human sequence antibody" includes antibodies having variable and constant regions (if present) derived from human germline immunoglobulin sequences. The human sequence antibodies of the present technology can include amino acid residues not encoded by human germline immunoglobulin sequences (e.g., mutations introduced by random or site-specific mutagenesis in vitro or by somatic mutation in vivo). Such antibodies can be generated in non-human transgenic animals, e.g., as described in PCT Publication Nos. WO 01/14424 and WO 00/37504. However, the term "human sequence antibody," as used herein, is not intended to include antibodies in which CDR sequences derived from the germline of another mammalian species, such as a mouse, have been grafted onto human framework sequences (e.g., humanized antibodies).

As used herein, the term "humanized" forms of non-human (e.g., murine) antibodies are chimeric antibodies which contain minimal sequence derived from non-human immunoglobulin. For the most part, humanized antibodies are human immunoglobulins in which hypervariable region residues of the recipient are replaced by hypervariable region residues from a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance such as binding affinity. Generally, the humanized antibody comprises substantially all of at least one, and typically two, variable domains, in which all or substantially all of the hypervariable loops correspond to those of a non-human immunoglobulin and all or substantially all of the FR regions are those of a human immunoglobulin sequence although the FR regions may include one or more amino acid substitutions that improve binding affinity. The number of these amino acid substitutions in the FR are typically no more than 6 in the H chain, and in the L chain, no more than 3. The humanized antibody optionally also comprises at least a portion of an immunoglobulin constant region (Fe), typically that of a human immunoglobulin. For further details, see Jones, et al., Nature 321:522-525 (1986); Reichmann, et al., Nature 332:323-329 (1988); and Presta, Curr. Op. Struct. Bioi. 2:593- 596 (1992).

As used herein, the term "hypervariable region" refers to the amino acid residues of an antibody which are responsible for antigen-binding. The hypervariable region generally comprises amino acid residues from a "complementarity determining region" or "CDR" (e.g. around about residues 24-34 (LI), 50-56 (L2) and 89-97 (L3) in the VL, and around about 31- 35B (HI), 50-65 (H2) and 95-102 (H3) in the VH (Kabat, et al., Sequences of Proteins of Immunological Interest, 5th Ed. Public Health Service, National Institutes of Health, Bethesda, MD. (1991)) and/or those residues from a "hypervariable loop" (e.g. residues 26- 32 (LI), 50-52 (L2) and 91-96 (L3) in the VL, and 26-32 (HI), 52A-55 (H2) and 96-101 (H3) in the VH (Chothia and Lesk J. Mol. Bioi. I96:90I-9I7 (1987)).

As used herein, "gene therapy" refers to the therapeutic delivery of nucleic acids into a patient's cells as a drug to treat disease. The delivery of DNA into cells can be accomplished by multiple methods. The two major classes are recombinant viruses (sometimes called biological nanoparticles or viral vectors) and naked DNA or DNA complexes (non-viral methods).

The term "halo" or "halogen" means fluoro, chloro, bromo, or iodo. Preferred halo groups are fluoro and chloro.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoro methyl, 1,1,1-trifluoroethyl and the like. Cx-Cy-haloalkyl and Cx-Cy-alkyl are alkyl groups which comprise x to y carbon atoms. Preferred haloalkyl groups are difluoromethyl and trifluoromethyl.

Where at least one carbon atom in an aryl group is replaced with a heteroatom, the resultant ring is referred to herein as a heteroaryl ring.

The term "heteroalkyl" means an alkyl group as defined above in which one or more carbon atoms are replaced by a heteroatom selected from oxygen, nitrogen and sulfur atoms. In heteroalkyl groups, the heteroatoms are linked along the alkyl chain only to carbon atoms, i.e. each heteroatom is separated from any other heteroatom by at least one carbon atom. However, the nitrogen and sulphur heteroatoms may optionally be oxidised and the nitrogen heteroatoms may optionally be quaternised. A heteroalkyl is bonded to another group or molecule only through a carbon atom, i.e. the bonding atom is not selected from the heteroatoms included in the heteroalkyl group.

The term "heteroaryl" as used herein by itself or as part of another group refers but is not limited to 5 to 12 carbon-atom aromatic rings or ring systems containing 1 to 2 rings which are fused together or linked covalently, typically containing 5 to 6 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2, 1 -b] [1 ,3] thiazolyl, thieno [3 ,2-b] furanyl, thieno [3 ,2-b] thiophenyl, thieno[2,3-d][I,3]thiazolyl, thieno[2,3-d]imidazolyl, tetrazolo[I,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2- benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3- benzoxadiazolyl, 1 ,2,3-benzothiadiazolyl, 2, 1 ,3-benzothiadiazolyl, thienopyridinyl, purinyl, imidazo[I,2-a]pyridinyl, 6-oxo-pyridazin-I(6H)-yl, 2- oxopyridin-I(2H)-yl, 6-oxo-pyridazin-I(6H)-yl, 2-oxopyridin-I(2H)-yl, 1,3- benzodioxolyl, quinolinyl, isoquinolinyl, cinnolinyl, quinazolinyl, quinoxalinyl.

Where at least one carbon atom in a cycloalkyl group is replaced with a heteroatom, the resultant ring is referred to herein as "heterocycloalkyl" or "heterocyclyl".

The terms "heterocyclyl", "heterocycloalkyl" or "heterocyclo" as used herein by itself or as part of another group refer to non-aromatic, fully saturated or partially unsaturated cyclic groups (for example, 3 to 7 member monocyclic, 7 to 11 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen, oxygen and/or sulfur atoms, where the nitrogen and sulfur heteroatoms may optionally be oxidized and the nitrogen heteroatoms may optionally be quaternized. Any of the carbon atoms of the heterocyclic group may be substituted by oxo (for example piperidone, pyrrolidinone). The heterocyclic group may be attached at any heteroatom or carbon atom of the ring or ring system, where valence allows. The rings of multi- ring heterocycles may be fused, bridged and/or joined through one or more spiro atoms. Non limiting exemplary heterocyclic groups include oxetanyl, piperidinyl, azetidinyl, 2-imidazolinyl, pyrazolidinyl imidazolidinyl, isoxazolinyl, oxazolidinyl, isoxazolidinyl, thiazolidinyl, isothiazolidinyl, piperidinyl, 3H-indolyl, indolinyl, isoindolinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, 3-dioxolanyl, 1,4-dioxanyl, 2,5-dioximidazolidinyl, 2- oxopiperidinyl, 2-oxopyrrolodinyl, indolinyl, tetrahydropyranyl, tetrahydrofuranyl, tetrahydroquinolinyl, tetrahydroisoquinolin- 1-yl, tetrahydroisoquinolin-2-yl, tetrahydroisoquinolin-3-yl, tetrahydroisoquinolin-4-yl, thiomorpholin-4-yl, thiomorpholin-4-ylsulf oxide, thiomorpholin-4-ylsulfone, 1,3- dioxolanyl, 1,4-oxathianyl, IH-pyrrolizinyl, tetrahydro-I,I-dioxothiophenyl, N- formylpiperazinyl, and morpholin-4-yl.

The term "non-proteinogenic amino acid" as used herein refers to an amino acid not naturally encoded or found in the genetic code of living organism. Non limiting examples of Non-proteinogenic amino acid are ornithine, citrulline, argininosuccinate, homoserine, homocysteine, cysteine-sulfinic acid, 2-aminomuconic acid, δ-aminolevulinic acid, β-alanine, cystathionine, γ-aminobutyrate, DOPA, 5-hydroxytryptophan, D-serine, ibotenic acid, α-aminobutyrate, 2-aminoisobutyrate, D-leucine, D-valine, D-alanine or D-glutamate .

The term "proteinogenic amino acid" as used herein refers to an amino acid that is incorporated into proteins during translation of messenger RNA by ribosomes in living organisms, i.e. Alanine (ALA), Arginine (ARG), Asparagine (ASN), Aspartate (ASP), Cysteine (CYS), Glutamate (glutamic acid) (GLU), Glutamine (GLN), Glycine (GLY), Histidine (HIS), Isoleucine (ILE), Leucine (LEU), Lysine (LYS), Methionine (MET), Phenylalanine (PHE), Proline (PRO), Pyrrolysine (PYL), Selenocysteine (SEL), Serine (SER), Threonine (THR), Tryptophan (TRP), Tyrosine (TYR) or Valine (VAL).

The term "prodrug" as used herein means the pharmacologically acceptable derivatives of compounds of formula (I) such as esters whose in vivo biotransformation product is the active drug. Prodrugs are characterized by increased bio-availability and are readily metabolized into the active compounds in vivo. Suitable prodrugs for the purpose of the invention include carboxylic esters, in particular alkyl esters, aryl esters, acyloxyalkyl esters, and dioxolene carboxylic esters; ascorbic acid esters.

The term "substituent" or "substituted" means that a hydrogen radical on a compound or group is replaced by any desired group which is substantially stable under the reaction conditions in an unprotected form or when protected by a protecting group. Examples of preferred substituents include, without being limited to, halogen (chloro, iodo, bromo, or fluoro); alkyl; alkenyl; alkynyl, as described above; hydroxy; alkoxy; nitro; thiol; thioether; imine; cyano; amido; phosphonato; phosphine; carboxyl; thiocarbonyl; sulfonyl; sulfonamide; ketone; aldehyde; ester; oxygen (-O); haloalkyl (e.g., trifluoromethyl); cycloalkyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic (e.g., pyrrolidinyl, piperidinyl, piperazinyl, morpholinyl, or thiazinyl), monocyclic or fused or non-fused polycyclic aryl or heteroaryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); CO2CH3; CONH2; OCH2CONH2; NH2; SO2NH2; OCHF2; CF3; OCF3; and such moieties may also be optionally substituted by a fused-ring structure or bridge, for example -OCH2O-. These substituents may optionally be further substituted with a substituent selected from such groups. In certain embodiments, the term "substituent" or the adjective "substituted" refers to a substituent selected from the group consisting of an alkyl, an alkenyl, an alkynyl, an cycloalkyl, an cycloalkenyl, a heterocycloalkyl, an aryl, a heteroaryl, an arylalkyl, a heteroarylalkyl, a haloalkyl, -C(O)NR11R12, -NR13C(O)R14, a halo, -OR13, cyano, nitro, a haloalkoxy, -C(O)R13, -NR11R12, -SR13, -C(O)OR13, -OC(O)R13, -NR13C(O)NR11R12, -OC(O)NR11R12, -NR13C(O)OR14, -S(O)rR13, -NR13S(O)rR14, -OS(O)rR14, S(O)rNR11R12, -O, -S, and -N-R13, wherein r is 1 or 2; R11 and R12, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl; or R11 and R12 taken together with the nitrogen to which they are attached is optionally substituted heterocycloalkyl or optionally substituted heteroaryl; and R13 and R14 for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocycloalkyl, an optionally substituted aryl, an optionally substituted heteroaryl, an optionally substituted arylalkyl, or an optionally substituted heteroarylalkyl. In certain embodiments, the term "substituent" or the adjective "substituted" refers to a solubilizing group.

The term "active ingredient" or "therapeutic ingredient" refers to a molecule or a substance whose administration to a subject slows down or stops the progression, aggravation, or deterioration of one or more symptoms of a disease, or condition; alleviates the symptoms of a disease or condition; cures a disease or condition. According to one embodiment, the therapeutic ingredient is a small molecule, either natural or synthetic. According to another the therapeutic ingredient is a biological molecule such as for example an oligonucleotide, a siRNA, a miRNA, a DNA fragment, an aptamer, an antibody and the like.

By "pharmaceutically acceptable" is meant that the ingredients of a pharmaceutical composition are compatible with each other and not deleterious to the patient thereof.

The term "pharmaceutically acceptable excipient" or "pharmaceutical vehicle" refers to an inert medium or carrier used as a solvent or diluent in which the pharmaceutically active agent is formulated and/or administered, and which does not produce an adverse, allergic or other reaction when administered to an animal, preferably a human being. This includes all solvents, dispersion media, coatings, antibacterial and antifungal agents, isotonic agents, absorption retardants and other similar ingredients. For human administration, preparations must meet standards of sterility, general safety and purity as required by regulatory agencies such as the FDA or EMA. For the purposes of the invention, "pharmaceutically acceptable excipient" includes all pharmaceutically acceptable excipients as well as all pharmaceutically acceptable carriers, diluents, and/or adjuvants.

The term "pharmaceutically acceptable salts" include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts.

Hemisalts of acids and bases may also be formed, for example, hemisulphate and hemicalcium salts.

Pharmaceutically acceptable salts of compounds of Formula (I) may be prepared by one or more of these methods:
(i) by reacting the compound of Formula (I) with the desired acid;
(ii) by reacting the compound of Formula (I) with the desired base;
(iii) by removing an acid- or base-labile protecting group from a suitable precursor of the compound of Formula (I) or by ring-opening a suitable cyclic precursor, e.g., a lactone or lactam, using the desired acid; and/or
(iv) by converting one salt of the compound of Formula (I) to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost non-ionized.

Although generally, with respect to the salts of the compounds of the invention, pharmaceutically acceptable salts are preferred, it should be noted that the invention in its broadest sense also included non-pharmaceutically acceptable salts, which may for example be used in the isolation and/or purification of the compounds of the invention. For example, salts formed with optically active acids or bases may be used to form diastereoisomeric salts that can facilitate the separation of optically active isomers of the compounds of Formula I above.

The term "solvate" is used herein to describe a molecular complex comprising a compound of the invention and contains stoichiometric or sub-stoichiometric amounts of one or more pharmaceutically acceptable solvent molecule, such as ethanol. The term 'hydrate' refers to when said solvent is water.

The term "administration", or a variant thereof (e.g., "administering"), means providing the active agent or active ingredient, alone or as part of a pharmaceutically acceptable composition, to the patient in whom/which the condition, symptom, or disease is to be treated or prevented.

The term "human" refers to a subject of both genders and at any stage of development (i.e., neonate, infant, juvenile, adolescent, adult).

The term "patient" refers to a warm-blooded animal, more preferably a human, who/which is awaiting the receipt of, or is receiving medical care or is/will be the object of a medical procedure.

The terms "treat", "treating" and "treatment", as used herein, are meant to include alleviating, attenuating or abrogating a condition or disease and/or its attendant symptoms.

The terms "prevent", "preventing" and "prevention", as used herein, refer to a method of delaying or precluding the onset of a condition or disease and/or its attendant symptoms, barring a patient from acquiring a condition or disease, or reducing a patient's risk of acquiring a condition or disease.

The term "therapeutically effective amount" (or more simply an "effective amount") as used herein means the amount of active agent or active ingredient that is sufficient to achieve the desired therapeutic or prophylactic effect in the patient to which/whom it is administered.

The bonds of an asymmetric carbon can be represented here using a solid triangle ( 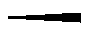 ), a dashed triangle ( 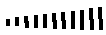 ) or a zigzag line ( 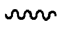 ).

### DETAILED DESCRIPTION OF THE INVENTION

The present invention thus relates to a compound of formula (I) : 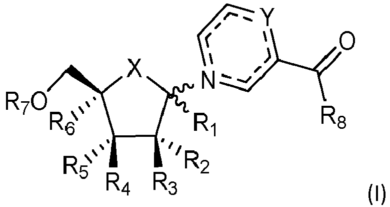 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 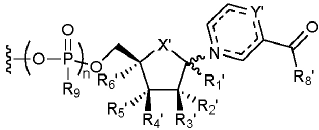 wherein n is an integer chosen amongst 1, 2 or 3; wherein:
   R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
   R11 is selected from C1-C10 alkyl, C3-C10 cycloalkyl, C5-C18 aryl, C1-C10 alkylaryl, substituted C5-C12 aryl, C1-C10 heteroalkyl, C3₋C10 heterocycloalkyle, C1-C10 haloalkyl, heteroaryl, -(CH2)nC(O)(C1-C15)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of R11a is independently selected from H and (C1-C6) alkyl and R11b is independently selected from OH, C1-C6 alkoxy, NH2, NH(C1-C6 alkyl) or N(C1-C6 alkyl)2 ;
   R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
   R13 and R14 are independently selected from H, C1-C8 alkyl and C1-C8 alkyl aryl;
   Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
   R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
   R9 and R10 together with the phosphorus atoms to which they are attached form a 6-membered ring wherein -R9-R10- represents-O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
   R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl;
   Y is selected from CH, CH2, C(CH3)2 and CCH3;
   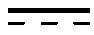 represents a single or double bond according to Y; and
   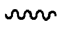 represents the alpha or beta anomer depending on the position of R1,
or a compound of formula (Ia) 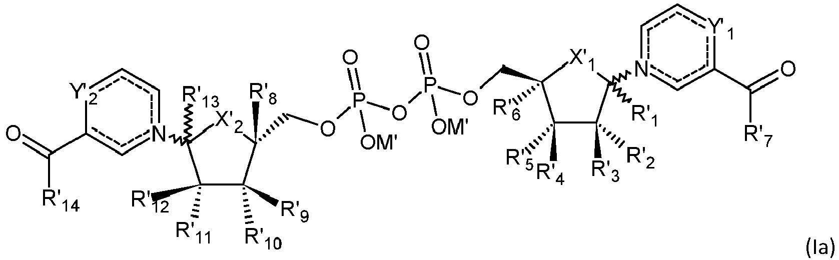 or pharmaceutically acceptable salts, hydrates, crystals, stereoisomers and/or solvates thereof, wherein:
X'1 and X'2 are independently selected from O, CH2, S, Se, CHF, CF2 and C=CH2;
R'1 and R'13 are independently selected from H, azido, cyano, C1-C8 alkyl, C1-C8 thio-alkyl, C1-C8 heteroalkyl and OR; wherein R is selected from H and C1-C8 alkyl;
R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid ;
R'6 and R'8 are independently selected from H, azido, cyano, C1-C8 alkyl and OR; wherein R is selected from H and C1-C8 alkyl;
R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
Y'1 and Y'2 are independently selected from CH, CH2, C(CH3)2 or CCH3;
M' is selected from H or a suitable counterion;
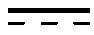 represents a single or a double bound depending on Y'1 and Y'2; and
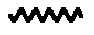 represents the alpha or beta anomer depending on the position of R'1 and R'13,
and their combinations for their use in the prevention and/or treatment of an alpha-synucleinopathy chosen amongst DLB or MSA.

Compounds of formula I and formula Ia are derivatives of nicotinamide mononucleotide. More specifically, compounds of formula I comprises NMN and its derivatives. Compounds of formula Ia are derivatives of nicotinamide mononucleotide (NMN).

Advantageously, X is selected from O, CH2 and S.

Advantageously, R1 is selected from hydrogen or OH. In one embodiment, R1 is hydrogen. In one embodiment, R1 is OH.

Advantageously, R2, R3, R4 and R5 are independently selected from hydrogen, halogen, hydroxyl, C1-C12 alkyl and OR; wherein R is as described herein above. In a preferred embodiment, R2, R3, R4 and R5 are independently selected from hydrogen, hydroxyl and OR; wherein R is as described herein above. In a more preferred embodiment R2, R3, R4 and R5 are independently selected from hydrogen or OH.

Advantageously, R2 and R3 are identical. In one embodiment, R2 and R3 are identical and represent OH. In one embodiment, R2 and R3 are identical and represent hydrogen.

Advantageously, R2 and R3 are different. In a preferred embodiment, R2 is hydrogen and R3 is OH. In a more preferred embodiment, R2 is OH and R3 is hydrogen.

Advantageously, R4 and R5 are identical. In one embodiment, R4 and R5 are identical and represent OH. In one embodiment, R4 and R5 are identical and represent hydrogen.

Advantageously, R2 and R3 are different. In a preferred embodiment, R4 is OH and R5 is hydrogen. In a more preferred embodiment, R4 is hydrogen and R5 is OH.

Advantageously, R3 and R4 are different. In one embodiment, R3 is OH and R4 is hydrogen. In one embodiment, R3 is hydrogen and R4 is OH.

Advantageously, R3 and R4 are identical. In a preferred embodiment, R3 and R4 are identical and represent OH. In a more preferred embodiment, R3 and R4 are identical and represent hydrogen.

Advantageously, R2 and R5 are different. In one embodiment, R2 is hydrogen and R5 is OH. In one embodiment, R2 is OH and R5 is hydrogen.

According to one embodiment, R2 and R5 are identical. In a preferred embodiment, R2 and R5 are identical and represent hydrogen. In a more preferred embodiment, R2 and R5 are identical and represent OH.

According to one embodiment, R6 is selected from hydrogen or OH. In one embodiment, R6 is OH. In a preferred embodiment, R6 is hydrogen.

Advantageously, R7 is selected from P(O)R9R10 or P(S)R9R10; wherein R9 and R10 are as described herein above. In a preferred embodiment, R7 is P(O)R9R10; wherein R9 and R10 are as described herein above. In a preferred embodiment, R7 is P(O)(OH)2.

Advantageously, R8 is selected from H, OR, NHR13 or NR13R14; wherein R13 and R14 are as described herein above. In a preferred embodiment, R8 is NHR13; wherein R13 and R14 are as described herein above.

Advantageously, Y is a CH or CH2. In one embodiment, Y is a CH. In one embodiment, Y is a CH2.

According to one preferred embodiment, compounds of formula (I) are those wherein X is an oxygen.

According to a preferred embodiment, the invention relates to compounds of general Formula (II): 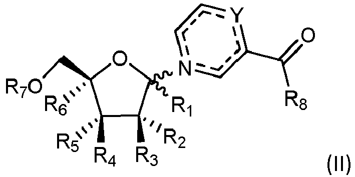 or a pharmaceutically acceptable salt or solvate thereof; wherein R1, R2, R3, R4, R5, R6, R7, R8, Y, 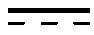 and 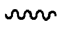 are as described herein above for compounds of formula (I).

Advantageously, preferred compounds of formula (I) are those wherein R1 is hydrogen.

Advantageously, preferred compounds of formula (I) are those wherein R2 is OH and R3 is hydrogen.

Advantageously, preferred compounds of formula (I) are those wherein R4 is hydrogen and R5 is OH.

Advantageously, preferred compounds of formula (I) are those wherein R3 and R4 are identical and represent hydrogen.

Advantageously, preferred compounds of formula (I) are those wherein R2 and R5 are identical and represent OH.

According to one embodiment, preferred compounds of formula (I) are those wherein R6 is hydrogen.

Advantageously, preferred compounds of formula (I) are those wherein R8 is NH2.

Advantageously, preferred compounds of formula (I) are those wherein Y is CH.

Advantageously, preferred compounds of formula (I) are those wherein Y is CH2.

Advantageously, preferred compounds of formula (I) are those wherein R7 is P(O)(OH)2.

Advantageously, the compound according to the invention is selected from compounds I-A to I-J from Table 1 below ci-dessous or a pharmaceutically acceptable salt or solvate thereof:

**[Table 1]**

| **Composés (anomères)** | **Structure** |
|---|---|
| I-A (beta) | 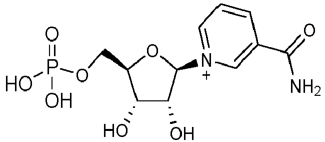 |
| I-B (alpha) | 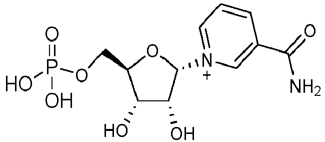 |
| I-C (beta) | 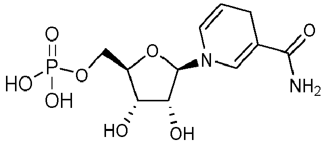 |
| I-D (alpha) | 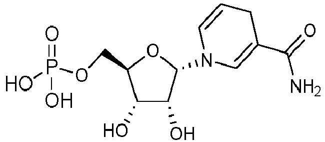 |
| I-E (beta) | 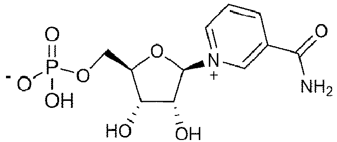 |
| I-F (alpha) | 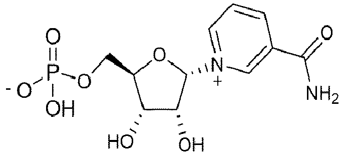 |
| I-G (beta) | 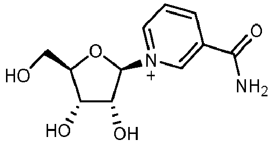 |
| I-H (alpha) | 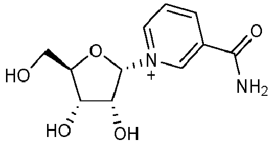 |
| I-I (beta) | 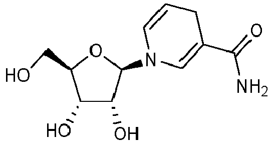 |
| I-J (alpha) | 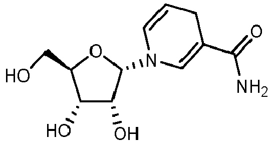 |

Advantageously, preferred compound of the invention are compounds I-A to I-F or a pharmaceutically acceptable salt or solvate thereof.

Advantageously, the pharmaceutically acceptable derivative is NMN-H of the following formula:

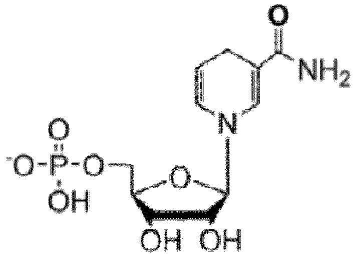

Advantageously, preferred compounds of general Formula Ia are those wherein X'1 and X'2 are independently selected from O, CH2, S.

Advantageously, R'7 and R'14 are independently selected from H, OR, NHR and NRR' wherein R and R' are independently selected from H, C1-C8 alkyl, C1-C8 alkyl aryl. According to one embodiment, R'7 and R'14 are NHR wherein R is selected from H, C1-C8 alkyl, C1-C8 alkyl aryl.

Advantageously, R'2, R'3, R'4, R'5, R'9, R'10, R'11, R'12 are independently selected from H, halogen, hydroxyl, C1-C12 alkyl and OR. According to a preferred embodiment, R'2, R'3, R'4, R'5, R'9, R'10, R'11, R'12 are independently selected from H, hydroxyl and OR, wherein R is as described herein above.

Advantageously, preferred compounds of general Formula Ia are those wherein, R'2, R'3, R'4, R'5, R'9, R'10, R'11, R'12 are independently selected from H and OH.

Advantageously, R'2 and R'3 are identical. According to one embodiment, R'2 and R'3 are identical and represent each a OH. According to one embodiment, R'2 and R'3 are identical and represent each hydrogen.

According to a preferred embodiment, R'2 and R'3 are different. According to a preferred embodiment, R'2 is hydrogen and R'3 is a OH. According to a more preferred embodiment, R'2 is a OH and R'3 is hydrogen.

Advantageously, R'4 and R'5 are identical. According to one embodiment, R'4 and R'5 are identical and represent each a OH. According to one embodiment, R'4 and R'5 are identical and represent each hydrogen.

According to a preferred embodiment, R'4 and R'5 are different. According to a preferred embodiment, R'4 is a OH and R'5 is hydrogen. According to a more preferred embodiment, R'4 is hydrogen and R'5 is a OH.

Advantageously, R'3 and R'4 are identical. According to one embodiment, R'3 and R'4 are identical and represent each a OH. According to one embodiment, R'3 and R'4 are identical and represent each hydrogen.

According to a preferred embodiment, R'3 and R'4 are different. According to a preferred embodiment, R'3 is a OH and R'4 is hydrogen. According to a more preferred embodiment, R'3 is hydrogen and R'4 is a OH

Advantageously, R'2 and R'5 are different. According to one embodiment, R'2 is hydrogen and R'5 is a OH. According to one embodiment, R'2 is a OH and R'5 is hydrogen.

According to a preferred embodiment, R'2 and R'5 are identical. According to a preferred embodiment, R'2 and R'5 are identical and represent each hydrogen. According to a more preferred embodiment, R'2 and R'5 are identical and represent each a OH.

Advantageously, R'9 and R'10 are identical. According to one embodiment, R'9 and R'10 are identical and represent each a OH. According to one embodiment, R'9 and R'10 are identical and represent each hydrogen.

According to a preferred embodiment, R'9 and R'10 are different. According to a preferred embodiment, R'9 is hydrogen and R'10 is a OH. According to a more preferred embodiment, R'9 is a OH and R'10 is hydrogen.

Advantageously, R'11 and R'12 are identical. According to one embodiment, R'11 and R'12 are identical and represent each a OH. According to one embodiment, R'11 and R'12 are identical and represent each hydrogen.

According to a preferred embodiment, R'11 and R'12 are different. According to a preferred embodiment, R'11 is a OH and R'12 is hydrogen. According to a more preferred embodiment, R'11 is hydrogen and R'12 is a OH.

Advantageously, R'10 and R'11 are different. According to one embodiment, R'10 is hydrogen and R'11 is a OH. According to one embodiment, R'10 is a OH and R'11 is hydrogen.

According to a preferred embodiment, R'10 and R'11 are identical. According to a preferred embodiment, R'10 and R'11 are identical and represent each a OH. According to a more preferred embodiment, R'10 and R'11 are identical and represent each hydrogen.

Advantageously, R'9 and R'12 are different. According to one embodiment, R'9 is hydrogen and R'12 is a OH. According to one embodiment, R'9 is a OH and R'12 is hydrogen.

According to a preferred embodiment, R'9 and R'12 are identical. According to a preferred embodiment, R'9 and R'12 are identical and represent each hydrogen. According to a more preferred embodiment, R'9 and R'12 are identical and represent each a OH.

Advantageously, Y'1 is CH. According to one embodiment, Y'1 is CH2.

Advantageously, Y'2 is CH. According to one embodiment, Y'2 is CH2.

Advantageously, X'1 and X'2 are different and are selected from the group as described above. According to one embodiment, X'1 and X'2 are identical and are selected from the group as described above.

Advantageously, preferred compounds of general Formula Ia are those wherein X'1 and X'2 each independently represents an Oxygen.

Advantageously, preferred compounds of general Formula Ia are those wherein X'1 and X'2 are identical and represent each an Oxygen.

Advantageously, R'7 and R'14 are different and are selected from the group as described above. According to one embodiment, R'7 and R'14 are identical and are selected from the group as described above.

Advantageously, preferred compounds of general Formula Ia are those wherein R'7 and R'14 each independently represents a NH2.

Advantageously, preferred compounds of general Formula Ia are those wherein R'7 and R'14 are identical and represent each a NH2.

Advantageously, R'1 and R'13 are different and are selected from the group as described above. According to one embodiment, R'1 and R'13 are identical and are selected from the group as described above.

Advantageously, preferred compounds of general Formula Ia are those wherein R'1 and R'13 each independently represents a hydrogen.

Advantageously, preferred compounds of general Formula Ia are those wherein R'1 and R'13 are identical and represent each a hydrogen.

Advantageously, R'6 and R'8 are different and are selected from the group as described above. According to one embodiment, R'6 and R'8 are identical and are selected from the group as described above.

Advantageously, preferred compounds of general Formula Ia are those wherein R'6 and R'8 each independently represents a hydrogen.

Advantageously, preferred compounds of general Formula Ia are those wherein R'6 and R'8 are identical and represent each a hydrogen.

Advantageously, R'3, R'4, R'10 and R'11 are different and are selected from the group as described above. According one embodiment, R'3, R'4, R'10 and R'11 are identical and are selected from the group as described above.

Advantageously, preferred compounds of general Formula Ia are those wherein R'3, R'4, R'10 and R'11 each independently represents a hydrogen.

Advantageously, preferred compounds of general Formula Ia are those wherein R'3, R'4, R'10, R'11 are identical and represent each a H.

Advantageously, R'2, R'5, R'9 and R'12 are different and are selected from the group as described above. According one embodiment, R'2, R'5, R'9 and R'12 are identical and are selected from the group as described above.

Advantageously, preferred compounds of general Formula Ia are those wherein R'2, R'5, R'9 and R'12 each independently represents a OH.

Advantageously, preferred compounds of general Formula Ia are those wherein R'2, R'5, R'9, R'12 are identical and represent each a OH.

Advantageously, Y'1 and Y'2 are different. According to a preferred embodiment, Y'1 and Y'2 are identical.

Advantageously, preferred compounds of general Formula Ia are those wherein Y'1 and Y'2 each independently represents a CH.

Advantageously, preferred compounds of general Formula Ia are those wherein Y'1 and Y'2 are identical and represent each a CH.

Advantageously, preferred compounds of general Formula Ia are those wherein Y'1 and Y'2 each independently represents a CH2.

Advantageously, preferred compounds of general Formula Ia are those wherein Y'1 and Y'2 are identical and represent each a CH2.

Advantageously, preferred compounds of the invention are compounds la-A to Ia-I, listed in table 2:

**[Table 2]**

| Compound | Structure |
|---|---|
| Ia-A (beta, beta) | 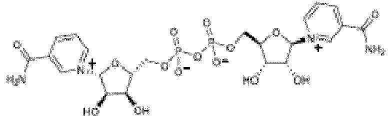 |
| Ia-B (beta, alpha) | 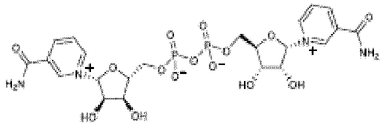 |
| Ia-C (alpha, alpha) | 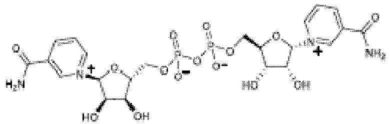 |
| la-D (beta, beta) | 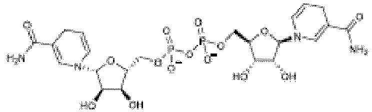 |
| Ia-E (beta, alpha) | 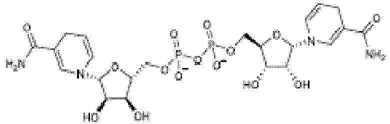 |
| Ia-F (alpha, alpha) | 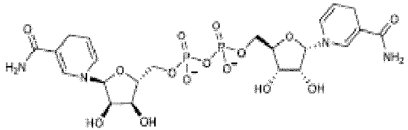 |
| Ia-G (beta, beta) | 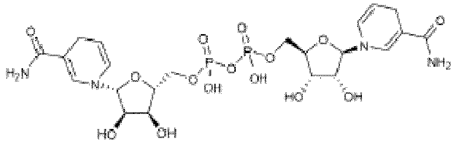 |
| la-H (beta, alpha) | 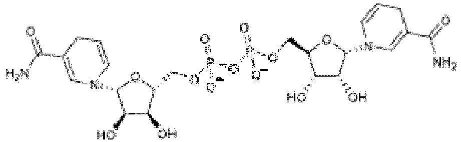 |
| Ia-I (alpha, alpha) | 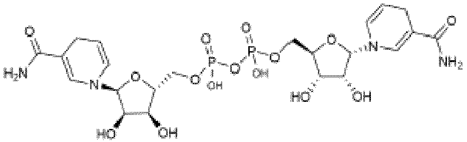 |

Advantageously, preferred compound of the invention is compound of formula la-C or la-F or Ia-I.

Advantageously, preferred compound of the invention is compound of formula la-B or la-E or la-H.

All references to compounds of Formula (I) or (Ia) include references to salts, solvates, multi component complexes and liquid crystals thereof. All references to compounds of Formula (I) or (Ia) include references to polymorphs and crystal habits thereof.

This invention also relates to the use of a compound of formula (I) and/or formula (Ia) as described herein in the treatment and/or prevention of an alpha-synucleinopathy such as dementia with Lewy bodies (DLB) and multiple system atrophy (MSA), preferably MSA, as described herein.

The invention also relates to a method of prevention and/or treatment of an alpha-synucleinopathy such as dementia with Lewy bodies (DLB) and multiple system atrophy (MSA), preferably MSA, as described herein.

This invention also relates to the use of a compound as described herein in the manufacture of a medicament for the treatment and/or prevention of an alpha-synucleinopathy such as dementia with Lewy bodies (DLB) and multiple system atrophy (MSA)preferably MSA, as described herein.

### Therapeutic use

Compounds of formula I and/or of formula Ia as well as compositions and kit of parts comprising the same may be used in the treatment and/or prevention of an alpha-synucleinopathy. Alpha-synucleinopathies wherein the invention can be particularly useful are dementia with Lewy bodies (DLB) and multiple system atrophy (MSA). Preferably, the alpha-synucleinopathy is MSA. According to the invention, the alpha-synucleinopathy is not Parkinson disease.

Indeed, the inventors have demonstrated that compounds of formula (I) and (Ia), unlike the conventional or experimental treatments of alpha-synucleinopathies so far tested, the use of the compounds of formula (I) and/or (Ia) according to the invention also provides good results on the alpha-synucleinopathies. Therefore, the invention provides an efficient treatment in order to reduce the symptoms of alpha-synucleinopathies, and preferably MSA, such as motor disorders, orthostatic hypotension and improve the autonomy of the patient and his ability to carry out the everyday tasks. In addition, the compounds of the invention are well tolerated and can be thus administered chronically, without involving any adverse events for the patients. The autonomy and ability to carry out every day task can be assessed by the Unified Multiple System Atrophy Rating Scale (UMSARS) questionary and/or the MSA-QoL questionary. The UMSARS questionary has been developed by a group of physician (G.K.Wenning et al., Movement Disorders, Vol. 19, No. 12, 2004, pp. 1391-1402, DOI: 10.1002/mds.20255). The improvement of quality of life can be assessed by the use of the MSA-QoL questionary (Anette Schrag et al, Movement Disorders, 2007 Dec;22(16):2332-8; https://doi.org/10.1002/mds.21649). These questionnaires are often used for the evaluation of the quality of life of PD patients. However, they are also useful for patients having an alpha-synucleinopathy.

The compounds of formula (I) or (Ia) as well as compositions and kit of part according to the invention are specifically useful to treat alpha-synucleinopathies such as DLB and MSA, as demonstrated by the inventors. Preferably, the alpha-synucleinopathy is multiple system atrophy (MSA).

In an embodiment, the compounds of formula (I) and/or compounds of formula (Ia) and compositions according to the invention are to be used in mammals, preferably humans. In an embodiment, the compounds of formula (I) or formula (Ia) and compositions according to the invention are to be used in male humans. In an embodiment, the compounds of formula (I) and/or compounds of formula (Ia) and compositions according to the invention are to be used in patients aged 18 or less than 18.

### Therapeutic combinations

Compounds of formula (I) and/or compounds of formula (Ia) as well as compositions comprising the same can be used in combinations with one or more further therapeutic ingredients conventionally used for the prevention and/or treatment of an alpha-synucleinopathy such as dementia with Lewy bodies (DLB) and multiple system atrophy (MSA), preferably MSA. Such combinations can be useful to prevent or treat various aspects of an alpha-synucleinopathy such as dementia with Lewy bodies (DLB) and multiple system atrophy (MSA). More specifically, the invention can be used in combination with the conventional treatments of an alpha-synucleinopathy, in order to prevent or treat dementia with Lewy bodies (DLB) or multiple system atrophy (MSA).

The at least one further therapeutic ingredient is chosen amongst a dopaminergic precursor, a monoamine oxidase type B inhibitor, an anticholinergic agent, a dopaminergic agonist, a noradrenaline precursor, a muscarinic antagonist, an alpha-blocker, a beta-adrenergic agonist, a corticoid, a phosphodiesterase inhibitor, a vasopressin V2 receptor agonist and their combinations.

Preferably, such at least one further therapeutic ingredient can be chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations. More preferably, the further therapeutic ingredient is levodopa (L-dopamine), with or without carbidopa.

The anticholinergic agent can be an antimuscarinic agent or a antinicotinic agent. The antimuscarinic agent can be chosen amongst clozapine, quietapine, atropine, benztropine, biperiden, chlorpherinamine, parotexine, dicyclomine, dimenhydrate, diphenhydramine, doxepin, doxylamine, glycopyrrolate, glycopyrronium, hyoscyamine, ipratropium, orphenadrine, oxitropium, oxybutynin, promethazine, propantheline bromide, scopolamine, solifenacin, tolterodine, tiotropium, tricyclic antidepressant, trihexyphenidyl, tropicamide, umeclidinium and their combinations, preferably diphenhydramine. The antinicotinic agent can be bupropion, dextromethorphan, doxacurium, hexamethonium, mecamylamine, tubocurarine and their combinations.

The acetylcholinesterase (ACE) inhibitor is chosen amongst benazepril, captopril, cilazapril, enalapril, fosinopril, imidapril, lisinopril, moexipril, perindopril, pyridostigmine, quinapril, ramipril, trandolapril, zofenopril and their combinations.

The tricyclic antidepressant can be amineptine, amitriptyline, amoxapine, butriptyline, clomipramine, desipramine, dibenzepin, dosulepin, doxepin, imipramine, iprindole, lofepramine, maprotiline, norclomipramine, northiaden, nortriptyline, opipramol, protriptyline, protriptyline, trimipramine, and their combinations. The benzodiazepine can be adinazolam, alprazolam, climazolam, clonazepam, clorazepate, diazepam, estazolam, flunitrazepam, flurazepam, halazepam, prazepam, loprazolam, lorazepam, lormetazepam, nimetazepam, nitrazepam, midazolam, oxazepam, temazepam, triazolam, and their combinations.

The dopaminergic agent can be a dopamine agonist, a dopamine antagonist, a dopamine transporter (DAT) inhibitor, a vesicular monoamine transporter (VMAT) inhibitor, a releasing agent, an enzyme inhibitor, and their combination.

The dopamine agonist can be chosen amongst:
- adamantanes: amantadine, memantine, rimantadine; and/or
- aminotetralins: 7-OH-DPAT, 8-OH-PBZI, rotigotine, UH-232; and/or
- benzazepines: 6-BR-APB, fenoldopam, SKF-38,393, SKF-77,434, SKF-81,297, SKF-82,958, SKF-83,959; and/or
- ergolines: bromocriptine, cabergoline, dihydroergocryptine, lisuride, lysergic acid diethylamide (LSD), pergolide; and/or
- dihydrexidine derivatives: 2-OH-NPA, A-86,929, ciladopa, dihydrexidine, dinapsoline, dinoxyline, doxanthrine: and/or
- A-68,930, A-77,636, A-412,997, ABT-670, ABT-724, aplindore, apomorphine, aripiprazole, bifeprunox, BP-897, CY-208,243, dizocilpine, etilevodopa, flibanserin, ketamine, melevodopa, modafinil, pardoprunox, phencyclidine, PD-128,907, PD-168,077, PF-219,061, piribedil, pramipexole, propylnorapomorphine, pukateine, quinagolide, quinelorane, quinpirole, RDS-127, RO10-5824, ropinirole, rotigotine, roxindole, salvinorin A, SKF-89,145, sumanirole, terguride, umespirone, WAY-100,635.

The dopamine antagonist can be chosen amongst:
- typical antipsychotics such as acepromazine, azaperone, benperidol, bromperidol, clopenthixol, chlorpromazine, chlorprothixene, droperidol, flupentixol, fluphenazine, fluspirilene, haloperidol, loxapine, mesoridazine, methotrimeprazine, nemonapride, penfluridol, perazine, periciazine, perphenazine, pimozide, prochlorperazine, promazine, sulforidazine, sulpiride, sultopride, thioridazine, thiothixene, trifluoperazine, triflupromazine, trifluperidol, zuclopenthixol; and/or

- atypical antipsychotics such as amisulpride, asenapine, blonanserin, cariprazine, carpipramine, clocapramine, clozapine, gevotroline, iloperidone, lurasidone, melperone, molindone, mosapramine, ocaperidone, olanzapine, paliperidone, perospirone, piquindone, quetiapine, remoxipride, risperidone, sertindole, tiospirone, ziprasidone, zotepine; and/or
- antiemetics such as AS-8112, alizapride, bromopride, clebopride, domperidone, metoclopramide, thiethylperazine; and/or
- amoxapine, buspirone, butaclamol, ecopipam, n-ethoxycarbonyl-2-ethoxy-1,2-dihydroquinoline (EEDQ); eticlopride, fananserin, L-745,870, nafadotride, nuciferine, PNU-99,194, raclopride, sarizotan, SB-277,011-A, SCH-23,390, SKF-83,566, SKF-83,959, sonepiprazole, spiperone, spiroxatrine, stepholidine, tetrahydropalmatine, tiapride, UH-232, yohimbine.

The dopamine transporter (DAT) inhibitor can be chosen amongst:
- piperazine such as DBL-583, GBR-12,935, nefazodone, vanoxerine, and/or
- piperidine such as 1-(1-(1-Benzothiophen-2-yl)cyclohexyl)piperidine (BTCP), desoxypipradrol, dextromethylphenidate, difemetorex, ethylphenidate, methylnaphthidate, isopropylphenidae, methylphenidate, phencyclidine, pipradrol; and/or
- pyrrolidine such as diphenylprolinol, methylenedioxypyrovalerone (mdpv), naphyrone, prolintane, pyrovalerone; and/or
- tropanes such as β-(4'-Chloropheny|)-2β-(3'-phenylisoxazol-5'-yl)tropane (β-CPPIT), altropane, brasofensine , WIN 35428 (β-CFT), cocaine, dichloropane , difluoropine, N-(2'-Fluoroethyl-)-3β-(4'-chlorophenyl)-2β-(3'-phenylisoxazol-5'-yl)nortropane (FE-β-CPPIT), N-(3'-Fluoropropyl-)-3β-(4'-chlorophenyl)-2β-(3'-phenylisoxazol-5'-yl)nortropane (FP-β-CPPIT), loflupane (123I), lometopane, RTI-112, RTI-113, RTI-121, RTI-126, RTI-150, RTI-177, RTI-229, RTI-336 , tenocyclidine, tesofensine, troparil, tropoxane, 2P-Propanoyl-3P-(4-tolyl)-tropane (WF-11), 2β-Propanoyl-3β-(2-naphthyl)-tropane (WF-23), 2-Propanoyl-3-(4-isopropylphenyl)-tropane (WF-31), 2α-(Propanoyl)-3β-(2-(6-methoxynaphthyl))-tropane (WF-33); and/or
- others such as adrafinil, armodafinil, amfonelic acid, amineptine, benzatropine (benztropine), Bromantane, 2-Butyl-3-(p-tolyl)quinuclidine (BTQ), BTS-74,398, bupropion (amfebutamone), ciclazindol, diclofensine, dimethocaine, diphenylpyraline, dizocilpine, DOV-102,677, DOV-21,947 , DOV-216,303, etybenzatropine (ethylbenztropine), EXP-561 , fencamine , fencamfamine , fezolamine, GYKI-52,895, hydrafinil, indatraline, ketamine, lefetamine, levophacetoperane, LR-5182, manifaxine, mazindol, medifoxamine, mesocarb, modafinil, nefopam, nomifensine, NS-2359, 0-2172, pridefrine, propylamphetamine, radafaxine , SEP-225,289, SEP-227,162, sertraline, sibutramine, tametraline, tripelennamine.

The vesicular monoamine transporter (VMAT) inhibitor can be deserpidine, ibogaine, reserpine and/or tetrabenazine.

The releasing agent can be chosen amongst:
- morpholine such as fenbutrazate, morazone, phendimetrazine, phenmetrazine; and/or
- oxazoline such as 4-methylaminorex (4-mar, 4-max), aminorex, clominorex, cyclazodone, fenozolone, fluminorex, pemoline, thozalinone; and/or
- phenethylamines (also amphetamines, cathinones, phentermines, etc.) such as 2-hydroxyphenethylamine (2-OH-PEA), 4-chlorophenylisobutylamine (4-CAB), 4-methylamphetamine (4-MA), 4-methylmethamphetamine (4-MMA), alfetamine, amfecloral, amfepentorex, amfepramone, amphetamine (dextroamphetamine, levoamphetamine), amphetaminil, β-methylphenethylamine, benzodioxolylbutanamine (bdb), benzodioxolylhydroxybutanamine, benzphetamine, buphedrone, butylone, cathine, cathinone, clobenzorex, clortermine, d-deprenyl, dimethoxyamphetamine (dma), dimethoxymethamphetamine (DMMA), dimethylamphetamine, dimethylcathinone (dimethylpropion, metamfepramone), ethcathinone (ethylpropion), ethylamphetamine, ethylbenzodioxolylbutanamine (EBDB), ethylone, famprofazone, fenethylline, fenproporex, flephedrone, fludorex, furfenorex, hordenine, lophophine (homomyristicylamine), mefenorex, mephedrone, methamphetamine (desoxyephedrine, methedrine; dextromethamphetamine, levomethamphetamine), methcathinone (methylpropion), methedrone, methoxymethylenedioxyamphetamine (MMDA), methoxymethylenedioxymethamphetamine (mmdma), methylbenzodioxolylbutanamine (MBDB), methylenedioxyamphetamine (mda, tenamfetamine), methylenedioxyethylamphetamine (MDEA), methylenedioxyhydroxyamphetamine, methylenedioxymethamphetamine, methylenedioxymethylphenethylamine, methylenedioxyphenethylamine, methylone, ortetamine, parabromoamphetamine, parachloroamphetamine (pca), parafluoroamphetamine, parafluoromethamphetamine, parahydroxyamphetamine, paraiodoamphetamine, paredrine (norpholedrine, oxamphetamine), phenethylamine, pholedrine, phenpromethamine, prenylamine, propylamphetamine, tiflorex (flutiorex), tyramine, xylopropamine, zylofuramine
- piperazine such as 2,5-dimethoxy-4-bromobenzylpiperazine, benzylpiperazine, methoxyphenylpiperazine (meopp, paraperazine), methylbenzylpiperazine (MBZP), methylenedioxybenzylpiperazine (MDBZP, piperonylpiperazine); and/or
- others such as 2-amino-1,2-dihydronaphthalene, 2-aminoindane, 2-aminotetralin, 4-benzylpiperidine, 5-iodo-2-aminoindane, clofenciclan, cyclopentamine, cypenamine, cyprodenate, feprosidnine, gilutensin, heptaminol, hexacyclonate, indanylaminopropane, indanorex, isometheptene, methylhexanamine, naphthylaminopropane, octodrine, phthalimidopropiophenone, propylhexedrine (levopropylhexedrine), tuaminoheptane (tuamine).

The enzyme inhibitors can be a phenylalanine hydroxylase inhibitor such as 3,4-dihydroxystyrene, a tyrosine hydroxylase inhibitors such as 3-iodotyrosine, aquayamycin, bulbocapnine, metirosine and/or oudenone, an aromatic L-amino acid decarboxylase (DOPA) inhibitor such as benserazide, carbidopa, genistein, methyldopa, a monoamine oxidase (MAO) inhibitor, a catechol-O-methyl transferase (COMT) inhibitor such as entacapone, nitecapone, opicapone, tolcapone, a dopamine beta hydroxylase inhibitor such as bupicomide, disulfiram, dopastin, fusaric acid, nepicastat, phenopicolinic acid and tropolone, and their combinations.

Compounds and compositions according to the invention can be administered with the further therapeutic ingredient simultaneously, separately, or sequentially. By "simultaneously" is meant that two agents are administered concurrently. By "separately" is meant that the gap between the administration of the first agent and that of the second is substantial. By "sequentially" is meant that the two agents are administered one after the other within a time frame such that they are both available to act therapeutically within the same time frame. The optimum time interval between administering the two agents will vary depending on the precise nature of the method for delivering the compounds or compositions of the invention.

### Composition

The present invention also relates to a composition comprising compound of formula I and/or compound of formula Ia as described hereinabove and at least one pharmaceutically acceptable excipient for its use in the prevention and/or treatment of an alpha-synucleinopathy such as dementia with Lewy bodies (DLB) and multiple system atrophy (MSA), preferably MSA, as described herein.

In one embodiment, the composition according to the invention is a pharmaceutical composition. In another embodiment, the composition according to the invention is a dietary supplement. Preferably, the composition according to the invention is a pharmaceutical composition.

In one embodiment, the pharmaceutical composition according to the invention may be used in combination with at least one further therapeutic ingredient as described herein. In another embodiment, the pharmaceutical composition according to the invention comprises as least one further therapeutic ingredient as described herein.

The compositions according to the invention may be formulated with carriers, excipients and diluents which are suitable in themselves for these formulations, such as lactose, dextrose, saccharose, sorbitol, mannitol, starches, acacia gum, calcium phosphate, alginates, tragacanth gum, gelatine, calcium silicate, microcrystalline cellulose, polyvinylpyrrolidone, polyethylene glycol, cellulose, water (sterile), methyl cellulose, methyl and propyl hydroxybenzoates, talc, magnesium stearate, edible oils, vegetable and mineral oils or suitable mixtures thereof. The formulations may possibly contain other substances that are commonly used in pharmaceutical formulations, such as lubricants, wetting agents, emulsifying and suspending agents, dispersing agents, disintegrants, fillers, preservatives, sweeteners, flavourings, flow regulators, mould release agents, etc. The compositions can also be formulated to allow a rapid, prolonged or delayed release of the active compound(s) they contain.

The compositions according to the invention are preferably in the form of unit doses and may be packaged in an appropriate manner, for example in a box, blister pack, bottle, sachet, ampoule or in any other suitable single-dose or multiple-dose carrier or container (which may be properly labelled); optionally with one or more leaflet(s) containing product information and/or instructions for use.

Compositions of the invention can be prepared by any method known by the skilled in the art.

### Methods of administration

The compounds of formula (I) and formula (Ia) as well as compositions of the invention as described herein, may be administered by oral, parenteral (e.g., intramuscular, intraperitoneal, intravenous, intracerebroventricular, intracisternal injection or infusion, subcutaneous injection, or implant), by inhalation spray, nasal, rectal, sublingual, or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals, such as mice, rats, horses, cattle, sheep, dogs, cats, monkeys, etc., the compounds of the invention are effective for use in humans. The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The compositions may be in a form suitable for oral use, for example, as tablets, troches, lozenges, aqueous or oily suspensions, dispersible powders or granules, emulsions, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material, such as glyceryl monostearate or glyceryl distearate may be employed. They may also be coated by the techniques described in the U.S. Patents 4,256,108; 4,166,452; and 4,265,874 to form osmotic therapeutic tablets for control release. Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy- propylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol , such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin. Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavoring agents may be added to provide a palatable oral preparation. These compositions may be preserved by the addition of an anti-oxidant, such as ascorbic acid. Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Syrups and elixirs may be formulated with sweetening agents, for example glycerol, propylene glycol, sorbitol or sucrose. Such formulations may also contain a demulcent, a preservative and flavouring and colouring agents.

The compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids, such as oleic acid find use in the preparation of injectables. The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols. For topical use, creams, ointments, jellies, solutions or suspensions, etc., containing the compounds of the present invention are employed. (For purposes of this application, topical application shall include mouthwashes and gargles.)

An appropriate dosage level is generally about 0.01 to 500 mg per kg patient body weight per day which can be administered in single or multiple doses. Preferably, the dosage level is about 0.1 to about 350 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. For example, the dosage may comprise from 100mg/day to 5000mg/day, preferably from 500mg/day to 1000mg/day.

"Gastro-resistant" means a galenic form that does not dissolve in the stomach. Such dosage forms are delayed release, i.e. they have a coating or coating composition that is resistant to the acid pH of the stomach (pH<2) to dissolve in the intestine. The gastroresistant character is determined by following the test established by the European Pharmacopoeia. Briefly, the gastroresistant character of a capsule is measured in 0.1 M hydrochloric acid at 37°C as a disintegrating medium in a disintegrating apparatus. This medium mimics the physicochemical conditions of the stomach. The capsules are incubated in this medium for 1 hour. The capsule must show no signs of disintegration or cracks that could lead to a loss of contents. The capsule is then incubated for 1 hour in a phosphate buffer solution of pH 6.8 at 37°C, this solution mimics the conditions of the intestinal environment in accordance with the recommendations of the European Pharmacopoeia. The capsule must be completely disintegrated within one hour.

"Sublingual tablet" means a galenic form that is placed under the tongue so that the active principle is absorbed by the sublingual mucosa, and in particular by the ranin veins and arteries.

The galenic form of the composition according to the invention may also be immediate release: such a galenic form allows rapid absorption of NMN, its salts or derivative, and thus a reduced time of action. Immediate release galenic forms include dispersible, orodispersible, effervescent tablets and oral lyophilisates.

The galenic form of the composition according to the invention may also be delayed release. Dissolution and absorption of the compounds of formula (I) and/or formula (Ia) of the invention takes place in the intestine, which limits gastric irritation or degradation of the fragile active ingredients at acidic pH. These are mainly gastroresistant forms, i.e. the tablets or granules are covered with a polymeric film, insoluble in an acidic medium but permeable to water in an alkaline medium or of the lipidic type degraded by intestinal lipases.

The galenic form of the composition according to the invention may also be a prolonged and sequential release form. The forms with sequential release (release at precise time intervals) and prolonged release (continuous release of the active principle until exhaustion) promote the spreading of the release of the active principle over time in order to maintain an effective plasma concentration for a longer period of time in the patient's body. Such galenic forms make it possible to obtain relief of the patient's pain for a longer period of time and to space out the medication intake.

The mode of administration and the galenic form are determined by the person in the profession according to the anatomical location of the pain to be treated and the patient. Reference is made to the latest edition of Remington's Pharmaceutical Sciences.

Compounds of formula (I) and/or compounds of formula (Ia) as described herein and the composition according to the invention may be administered between 1 to 4 times per day, preferably once, twice or three times per day. Once or twice a day has been found suitable. Preferably, the compounds and compositions of the invention are to be taken before breakfast.

The duration of the treatment depends from the patent and is determined by the physician. It can be from one day to one year or even longer, preferably from one week to three months, more preferably from two weeks to six weeks. It will be understood, however, that the specific dose level and frequency of dosage and duration for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

### Kit of parts

The present invention also relates to a kit of parts comprising at least compound of formula I and/or compound of formula Ia or a composition of the invention as described hereinabove, and at least one further part.

In one embodiment, the at least one further part may be an information leaflet which may comprise information to the user and/or instructions for use. In one embodiment, the at least one further part may be at least one further therapeutic ingredient as set out above. Preferably, the at least one further part comprises an information leaflet and at least one further therapeutic ingredient as described herein.

The kit of parts may be used in the treatment and/or prevention of an alpha-synucleinopathy such as dementia with Lewy bodies (DLB) and multiple system atrophy (MSA), preferably MSA as described hereinabove.

### Synthesis of compounds of the invention

According to another aspect, the invention relates to a method for the preparation of the compound of Formula (I) as described above. Compounds of formula (I) can also be prepared according to the methods disclosed in international patent application WO 2017/079195 A1 and US patent US 10,611,790 B2.

In particular, the compounds of Formula (I) disclosed herein may be prepared as described below from substrates A-E. It shall be understood by a person skilled in the art that these schemes are in no way limiting and that variations may be made without departing from the spirit and scope of this invention.

The method can involve in a first step the mono-phosphorylation of a compound of formula (A), in the presence of phosphoryl chloride and a trialkyl phosphate, to yield the phosphorodichloridate of formula (B), 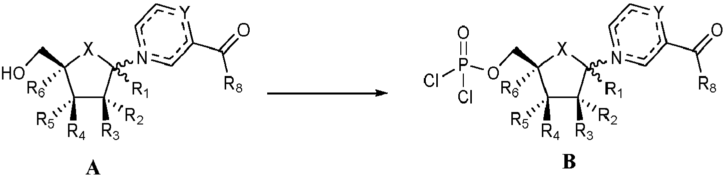 wherein X, R1, R2, R3, R4, R5, R6, R7, R8, Y, 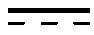 and 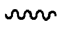 are as described herein above for compounds of formula (I).

In a second step, the phosphorodichloridate of formula (B) is hydrolysed to yield the phosphate of formula (C), 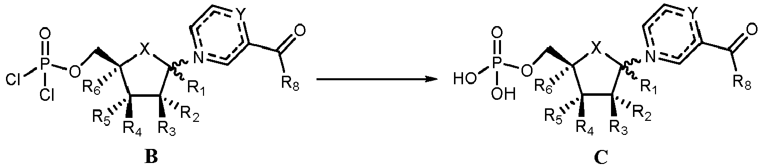 wherein X, R1, R2, R3, R4, R5, R6, R7, R8, Y, 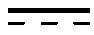 and 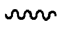 are as described herein above for compounds of formula (I).

The compound of formula (A) can be synthesized using various methods known to the person skilled in the art. According to one embodiment, the compound of formula (A) is synthesized by reacting the pentose of formula (D) with a nitrogen derivative of formula (E), wherein R, R2, R3, R4, R5, R6, R7, Y are as described above for compounds of formula (I), leading to the compound of formula (A-1) which is then selectively deprotected to give the compound of formula (A), 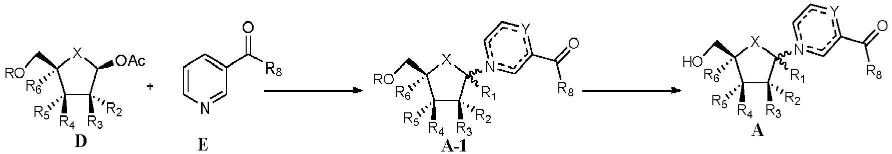 wherein X, R1, R2, R3, R4, R5, R6, R8, Y, 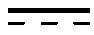 and 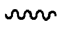 are as described herein above for compounds of formula (I).

R can be an appropriate protective group known to the skilled person in the art. In one embodiment, the protecting group is selected from triarylmethyls and/or silyls. Non-limiting examples of triarylmethyl include trityl, monomethoxytrityl, 4,4'-dimethoxytrityl and 4,4',4"-trimethoxytrityl. Non-limiting examples of silyl groups include trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

Any hydroxyl group can be attached to the pentose is protected by an appropriate protective group known to the person skilled in the art.

The choice and exchange of protective groups is the responsibility of the person skilled in the art. Protective groups can also be removed by methods well known to the skilled person, for example, with an acid (e.g. mineral or organic acid), base or fluoride source. According to a preferred embodiment, the nitrogen derivative of formula (E) is coupled to the pentose of formula (D) by a reaction in the presence of a Lewis acid leading to the compound of formula (A-1). Non-limiting examples of Lewis acids include TMSOTf, BF3.OEt2, TiCl4 and FeCl3.

The method of the present invention can further comprise a step of reducing the compound of formula (A) by various methods well known to the skilled person in the art, leading to the compound of formula (A') wherein is CH2 and R1, R2, R3, R4, R5, R6, R8, Y, 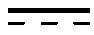 and 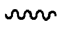 are as defined above for compounds of formula (I).

According to a specific embodiment, the present invention relates to a method for the preparation of the compounds of formula I-A to I-D.

In a first step, the nicotinamide of formula E is coupled to the ribose tetraacetate of formula D by a coupling reaction in the presence of a Lewis acid, resulting in the compound of formula A-1:

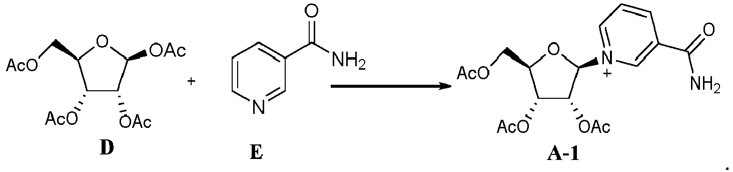

In a second step, an ammoniacal treatment of the compound of formula A-1 is carried out, leading to the compound of formula A-2:

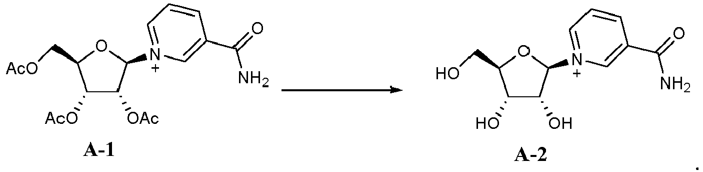

In a third step, the mono-phosphorylation of the compound of formula A-2, in the presence of phosphoryl chloride and a trialkyl phosphate, leads to the phosphorodichloridate of formula A-3:

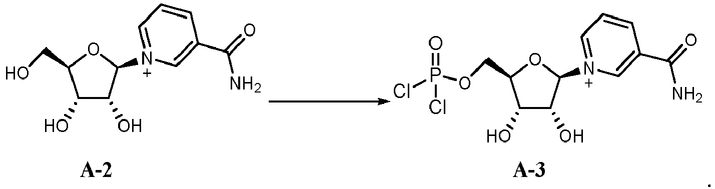

In a fourth step, the phosphorodichloridate of formula A-3 is hydrolysed to yield the compound of formula I-A:

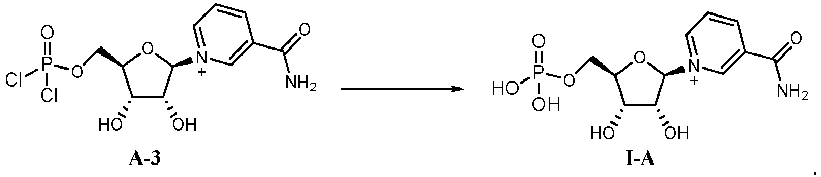

According to one embodiment, a step of reducing the compound of formula A-2 is carried out, leading to the compound of formula I-E of formula:

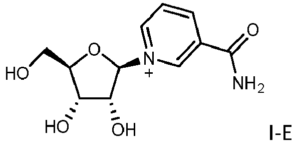

The compound of formula I-E is then monophosphorylated as described in step 4 and hydrolyzed to the compound of formula I-C.

In another aspect, the invention relates to a method for preparing compounds of formula Ia as described above.

In particular, compounds of formula Ia disclosed herein can be prepared as described below from substrates Xa-Xllla. It will be understood by one ordinary skilled in the art that these schemes are in no way limiting and that variations of detail can be made without departing from the spirit and scope of the present invention.

According to one embodiment, the invention relates to a method for preparing the compound of formula I described herein above.

The method first involves the mono-phosphorylation of a compound of formula Xa, in the presence of phosphoryl chloride in a trialkyl phosphate, to give the phophorodichloridate compound Xla, 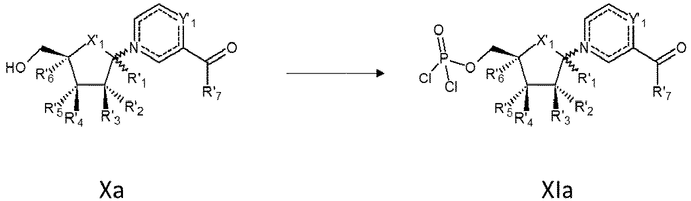 wherein X'1, R'1, R'2, R'3, R'4, R'5, R'6, R'7, Y'1, 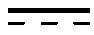 and 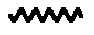 are as described herein for formula Ia.

In a second step the hydrolysis of the phophorodichloridate XIa obtained in the first step give the phosphate compound of formula Xlla, 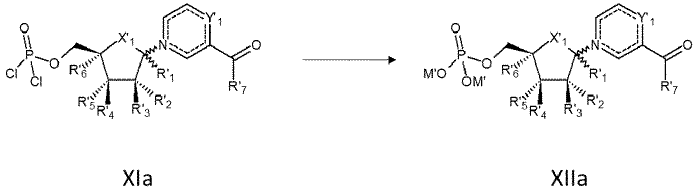 wherein X'1, R'1, R'2, R'3, R'4, R'5, R'6, R'7, Y'1, M', 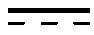 and 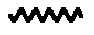 are as described herein for formula Ia.

The phosphate compound of formula Xlla obtained in the second step is then reacted, with a phophorodichloridate compound of formula XIIIa obtained as described in the first step, 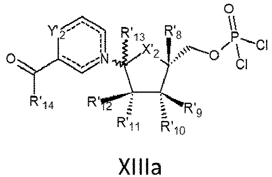 wherein X'2, R'8, R'9, R'10, R'11, R'12, R'13, R'14, Y'2, 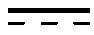 and 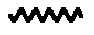 are as are as described herein for formula la, to give the compound of formula Ia as described herein.

According to one embodiment, the method of the invention further comprises a step of reducing the compound of formula la, using various methods known to those skilled in the art, to give the compound of formula I'a, wherein Y'1 and Y'2 are identical and represent each CH2 and wherein X'1, X'2, R'1, R'2, R'3, R'4, R'5, R'6, R'7, R'8, R'9, R'10, R'11, R'12, R'13, R'14, Y'1, Y'2, M', 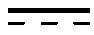 and 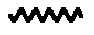 are as described herein for formula Ia.

According to another embodiment, the compound of formula Xa is synthesized using various methods known to those skilled in the art. According to one embodiment, the compound of formula Xa is synthesized in two steps by first reacting the pentose of formula XIVa with the nitrogenous derivatives of formula XVa, wherein R'1, R'1, R'2, R'3, R'4, R'5, R'6, R'7, Y'1 and R are as described herein for formula la, to give the compound of formula Xa-1, then selectively deprotected to give the compound of formula Xa. 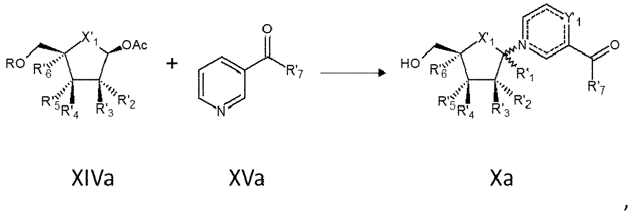 wherein X'1, R, R'1, R'2, R'3, R'4, R'5, R'6, R'7, Y'1, 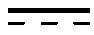 and 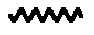 are as described herein for formula Ia.

According to one embodiment, R is an appropriate protecting group known to those skilled in the art. Examples of appropriate protecting group includes triarylmethyl and/or silyl groups. Non limiting examples of triarylmethyl includes trityl, monomethoxytrityl, 4,4'-dimethoxytrityl and 4,4',4"-trimethoxytrityl. Non limiting examples of silyl groups includes trimethylsilyl, tert-butyldimethylsilyl, triisopropylsilyl, tert-butyldiphenylsilyl, tri-iso-propylsilyloxymethyl and [2-(trimethylsilyl)ethoxy]methyl.

According to one embodiment, any hydroxy group attached to the pentose ring is protected with an appropriate protecting group known to those skilled in the art.

The selection and exchange of the protecting groups is within the skill to those skilled in the art. Any protecting groups can also be removed by methods known in the art, for example, with an acid (e.g., a mineral or an organic acid), a base or a fluoride source.

According to a preferred embodiment, the nitrogenous derivatives of formula XVa is added to the pentose XIVa via a coupling reaction in the presence of a Lewis acid to give the compound of formula Xa-1. Non limiting examples of suitable Lewis acid includes TMSOTf, BF3.OEt2, TiCI4 and FeCl3.

According to a specific embodiment, the invention relates to a method for preparing the compound of formula Villa, 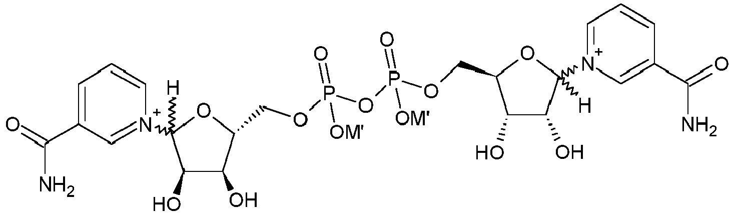 or pharmaceutically acceptable salts and/or solvates thereof.

In a first step, the nicotinamide of formula XVa, is added to the ribose tetraacetate XIVa, via a coupling reaction in the presence of a Lewis acid, to give the compound of formula Xa-1:

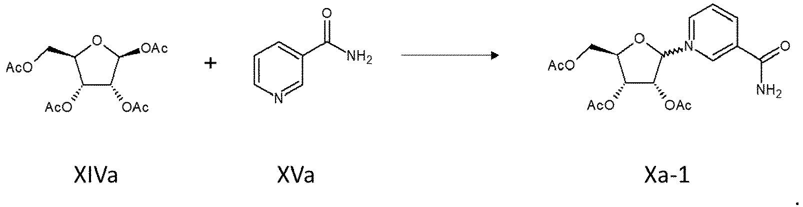

In a second step, an ammoniacal treatment of the compound of formula Xa-1 give the compound of formula Xa:

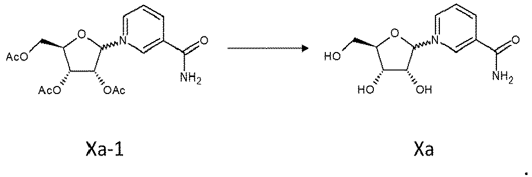

In a third step, the mono-phosphorylation of a compound of formula Xa, in the presence of phosphoryl chloride in a trialkyl phosphate, give the phophorodichloridate compound Xla:

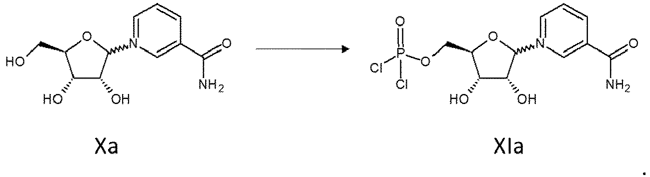

In a fourth step, the phophorodichloridate compound XIa obtained in the third step is partially hydrolyzed to give the phosphate compound of formula Xlla:

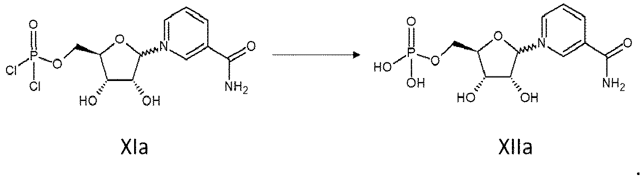

In a fifth step, the phosphate compound of formula Xlla obtained in the fourth step is then reacted, with the phophorodichloridate compound of formula XIa obtained as described in the third step, to give the compound of formula Villa.

According to another specific embodiment, the invention relates to a method for preparing the compound of formula IXa, 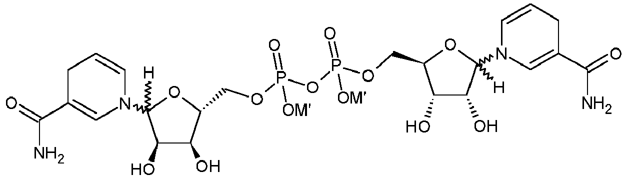 or pharmaceutically acceptable salts and/or solvates thereof.

According to one embodiment, the compound of formula IXa is obtained from the compound of formula Villa, previously synthesized as described above.

In this embodiment, the compound of formula IXa is obtained by reducing the compound of formula Villa, using a suitable reducing agent known to those skilled in the art, to give the compound of formula IXa.

### EXAM PLES

In the following description, the examples are provided to illustrate the present invention and are in no way intended to limit its scope.

### Example 1 - Administration to a 55 years old male patient having multiple system atrophy (MSA)

A 55-year-old Male presenting with MSA was given 125 mg NMN (beta) for 6 months as an oral tablet every morning. The patient exhibited low response to L-dopa treatment and orthostatic hypotension before treatment. He indicated difficulties to get out of bed in the mornings and feeling low and depressed during the day. Administration of NMN (beta) occurred once per day in the morning. The patient has taken the MSA-QoL questionary before the beginning of the study (D0) and after 6 months of treatment. The results are shown below.

**Table 3 - MSA-QoL questionary - D0:**

| **In the last 4 weeks, did you** | **No problem** | **Mild problem** | **Average problem** | **Problem marked** | **Extreme problem** | **NA** |
|---|---|---|---|---|---|---|
| 1. have difficulties to move? | x | | | | | |
| 2. have difficulty walking? | x | | | | | |
| 3. have balance difficulties? | | x | | | | |
| 4. have difficulty standing up without help? | x | | | | | |
| 5. have difficulties in speaking? | x | | | | | |
| 6. have difficulties swallowing food? | | | x | | | |
| 7. have too much saliva or do you drool? | x | | | | | |
| 8. have difficulties writing by hand? | | | x | | | |
| 9. have difficulties feeding yourself? | | x | | | | |
| 10.have difficulties in drinking liquids? | x | | | | | |
| 11.have difficulties getting dressed? | | x | | | | |
| 12.needed help to go to the toilets? | x | | | | | |
| 13.have to stop doing the things you used to like to do (e.g. your favorite hobbies)? | | x | | | | |
| 14.have difficulties with domestic activities (e.g. housework)? | | x | | | | |
| 15.have bladder problems? | | | | x | | |
| 16.have constipation problems? | | | x | | | |
| 17.have dizziness when getting up? | | x | | | | |
| 18.have a feeling of cold hands or cold feet? | | x | | | | |
| 19.have pain in the neck or shoulders? | | x | | | | |
| 20.have pain elsewhere (e.g. back, legs)? | x | | | | | |
| 21.have difficulties being comfortable at night? | | x | | | | |
| 22.have difficulty breathing at night? | x | | | | | |
| 23.have the feeling of being tired very quickly (without having made any effort)? | x | | | | | |
| 24.have a loss of energy? | | x | | | | |
| 25.have a slowdown in thinking? | | x | | | | |
| 26.have difficulties concentrating (e.g. reading or television)? | | x | | | | |
| 27.have the feeling of being frustrated? | | x | | | | |
| 28.have the feeling of being depressed? | | | x | | | |
| 29.have a loss of motivation? | | | x | | | |
| 30.have the feeling of being powerless? (in the face of events) | | | x | | | |
| 31.have concerns about the future? | | | x | | | |
| 32.have concerns about your family? | | x | | | | |
| 33.have the feeling of being abandoned or isolated? | | | x | | | |
| 34.have a lack of self-confidence in your relationship with others? | x | | | | | |
| 35.have the feeling that your role in your family or among your friends has changed? | | x | | | | |
| 36.have having difficulty seeing your friends? | x | | | | | |
| 37.have to give up your social activities (e.g. going out for meals or shows)? | x | | | | | |
| 38.have difficulties talking to friends about your illness? | x | | | | | |
| 39.felt embarrassed to talk to people? | x | | | | | |
| 40.have the feeling that life has become boring? | | | x | | | |

**Table 4 - MSA-QoL questionary - after 6 months of treatment:**

| **In the last 4 weeks do you have** | **No problem** | **Mild problem** | **Average problem** | **Problem marked** | **Extreme problem** | **NA** |
|---|---|---|---|---|---|---|
| 1. have difficulties to move? | x | | | | | |
| 2. have difficulty walking? | x | | | | | |
| 3. have balance difficulties? | x | | | | | |
| 4. have difficulty standing up without help? | x | | | | | |
| 5. have difficulties in speaking? | x | | | | | |
| 6. have difficulties swallowing food? | | | x | | | |
| 7. have too much saliva or do you drool? | x | | | | | |
| 8. have difficulties writing by hand? | | x | | | | |
| 9. have difficulties feeding yourself? | x | | | | | |
| 10.have difficulties in drinking liquids? | x | | | | | |
| 11.have difficulties getting dressed? | | x | | | | |
| 12.needed help to go to the toilets? | x | | | | | |
| 13.have to stop doing the things you used to like to do (e.g. your favorite hobbies)? | | x | | | | |
| 14.have difficulties with domestic activities (e.g. housework)? | x | | | | | |
| 15.have bladder problems? | | | | x | | |
| 16.have constipation problems? | | | x | | | |
| 17.have dizziness when getting up? | | x | | | | |
| 18.have a feeling of cold hands or cold feet? | x | | | | | |
| 19.have pain in the neck or shoulders? | | x | | | | |
| 20.have pain elsewhere (e.g. back, legs)? | x | | | | | |
| 21.have difficulties being comfortable at night? | | x | | | | |
| 22.have difficulty breathing at night? | x | | | | | |
| 23.have the feeling of being tired very quickly (without having made any effort)? | x | | | | | |
| 24.have a loss of energy? | x | | | | | |
| 25.have a slowdown in thinking? | | x | | | | |
| 26.have difficulties concentrating (e.g. reading or television)? | x | | | | | |
| 27.have the feeling of being frustrated? | x | | | | | |
| 28.have the feeling of being depressed? | | x | | | | |
| 29.have a loss of motivation? | | | x | | | |
| 30.have the feeling of being powerless? (in the face of events) | | x | | | | |
| 31.have concerns about the future? | | | x | | | |
| 32.have concerns about your family? | | x | | | | |
| 33.have the feeling of being abandoned or isolated? | | x | | | | |
| 34.have a lack of self-confidence in your relationship with others? | x | | | | | |
| 35.have the feeling that your role in your family or among your friends has changed? | x | | | | | |
| 36.have having difficulty seeing your friends? | x | | | | | |
| 37.have to give up your social activities (e.g. going out for meals or shows)? | x | | | | | |
| 38.have difficulties talking to friends about your illness? | x | | | | | |
| 39.felt embarrassed to talk to people? | x | | | | | |
| 40.have the feeling that life has become boring? | | | x | | | |

As it can be seen above, items 3, 8, 9, 14, 18, 24, 26-28, 30, 33 and 35 have been improved by the treatment in 6 months. Otherwise stated, the daily administration of beta-NMN to the MSA patient improved motricity and voluntary movements (item No.8, 9, 14). More noticeably, daily administration of NMN reduced postural hypotension (item No.3), a key symptom of MSA.

**Table 5 - UMSARS questionary - D0:**

| How many times did you experience one of the following situations during the last month? | | Never | Rarely | Sometimes | Often | Always or totally unable |
|---|---|---|---|---|---|---|
| 1 | Did you had any difficulties in the practice of your hobbies? | | | x | | |
| 2 | Have you had difficulty taking care of your home, for example: do-it-yourself, cleaning, cooking? | | | x | | |
| 3 | Did you have difficulty carrying shopping bags? | x | | | | |
| 4 | Did you have problems walking 1 km? | x | | | | |
| 5 | Did you have problems walking 100 m? | x | | | | |
| 6 | Did you have problems getting around your home as easily as you would have liked? | x | | | | |
| 7 | Have you had difficulty getting around in public places? | x | | | | |
| 8 | Did you need someone to accompany you on your outings? | x | | | | |
| 9 | Were you scared or worried about falling in public? | x | | | | |
| 10 | Have you been confined to your home more than you would have liked? | x | | | | |
| 11 | Did you have difficulty washing yourself? | x | | | | |
| 12 | Did you have difficulty getting dressed? | | x | | | |
| 13 | Have you had problems buttoning your clothes or tying your shoes? | | | x | | |
| 14 | Did you have problems writing legibly? | | | x | | |
| 15 | Did you have difficulty cutting the food? | | | x | | |
| 16 | Have you had difficulty holding a glass without spilling it? | | x | | | |
| 17 | Did you feel depressed? | | | | x | |
| 18 | Did you feel isolated and alone? | | | x | | |
| 19 | Did you feel on the verge of tears or did you cry? | | | x | | |
| 20 | Did you feel anger or bitterness? | | x | | | |
| 21 | Did you feel anxious? | | | x | | |
| 22 | Did you feel worried about your future? | | | | x | |
| 23 | Have you felt the need to hide your Parkinson's disease from others? | | x | | | |
| 24 | Have you avoided situations where you had to eat or drink in public? | x | | | | |
| 25 | Have you felt embarrassed in public because of your Parkinson's disease? | | x | | | |
| 26 | Did you feel worried about how others would react to you? | | | x | | |
| 27 | Have you had problems in your relationships with your loved ones? | | x | | | |
| 28 | Did you lack the support you needed from your spouse or partner? | | | x | | |
| 29 | Did you lack the support you needed from family or close friends? | | | x | | |
| 30 | Did you fall asleep during the day unexpectedly? | x | | | | |
| 31 | Have you had problems concentrating, for example while reading or watching television? | | x | | | |
| 32 | Did you feel that your memory was bad? | | | x | | |
| 33 | Did you have bad dreams or hallucinations? | | | x | | |
| 34 | Did you have difficulty speaking? | | x | | | |
| 35 | Have you felt unable to communicate normally with others? | | x | | | |
| 36 | Did you feel ignored by others? | | x | | | |
| 37 | Have you had painful muscle cramps or spasms? | | x | | | |
| 38 | Have you had pain or discomfort in your joints or body? | x | | | | |
| 39 | Have you had the unpleasant sensation of hot or cold? | x | | | | |

**Table 6 - UMSARS questionary - after 6 months of treatment:**

| How many times did you experience one of the following situations during the last month? | | Never | Rarely | Sometimes | Often | Always or totally unable |
|---|---|---|---|---|---|---|
| 1 | Did you had any difficulties in the practice of your hobbies? | | x | | | |
| 2 | Have you had difficulty taking care of your home, for example: do-it-yourself, cleaning, cooking? | | | x | | |
| 3 | Did you have difficulty carrying shopping bags? | x | | | | |
| 4 | Did you have problems walking 1 km? | x | | | | |
| 5 | Did you have problems walking 100 m? | x | | | | |
| 6 | Did you have problems getting around your home as easily as you would have liked? | x | | | | |
| 7 | Have you had difficulty getting around in public places? | x | | | | |
| 8 | Did you need someone to accompany you on your outings? | x | | | | |
| 9 | Were you scared or worried about falling in public? | x | | | | |
| 10 | Have you been confined to your home more than you would have liked? | x | | | | |
| 11 | Did you have difficulty washing yourself? | x | | | | |
| 12 | Did you have difficulty getting dressed? | | x | | | |
| 13 | Have you had problems buttoning your clothes or tying your shoes? | | x | | | |
| 14 | Did you have problems writing legibly? | | x | | | |
| 15 | Did you have difficulty cutting the food? | | | x | | |
| 16 | Have you had difficulty holding a glass without spilling it? | x | | | | |
| 17 | Did you feel depressed? | | | x | | |
| 18 | Did you feel isolated and alone? | | x | | | |
| 19 | Did you feel on the verge of tears or did you cry? | | | x | | |
| 20 | Did you feel anger or bitterness? | | x | | | |
| 21 | Did you feel anxious? | | | x | | |
| 22 | Did you feel worried about your future? | | | x | | |
| 23 | Have you felt the need to hide your Parkinson's disease from others? | | x | | | |
| 24 | Have you avoided situations where you had to eat or drink in public? | x | | | | |
| 25 | Have you felt embarrassed in public because of your Parkinson's disease? | | x | | | |
| 26 | Did you feel worried about how others would react to you? | | x | | | |
| 27 | Have you had problems in your relationships with your loved ones? | | x | | | |
| 28 | Did you lack the support you needed from your spouse or partner? | | x | | | |
| 29 | Did you lack the support you needed from family or close friends? | | x | | | |
| 30 | Did you fall asleep during the day unexpectedly? | x | | | | |
| 31 | Have you had problems concentrating, for example while reading or watching television? | | x | | | |
| 32 | Did you feel that your memory was bad? | | | x | | |
| 33 | Did you have bad dreams or hallucinations? | | | x | | |
| 34 | Did you have difficulty speaking? | x | | | | |
| 35 | Have you felt unable to communicate normally with others? | x | | | | |
| 36 | Did you feel ignored by others? | x | | | | |
| 37 | Have you had painful muscle cramps or spasms? | | x | | | |
| 38 | Have you had pain or discomfort in your joints or body? | x | | | | |
| 39 | Have you had the unpleasant sensation of hot or cold? | x | | | | |

Therefore, the administration of beta-NMN according to the invention improved items 1, 13, 14, 16-18, 22, 26, 28, 29, 34-36 of the UMSARS questionary. Notably, it appears that the daily administration of beta-NMN (compound IA) did not improve most of the clinical signs related to motricity apart from items 1, 13, 14 and 16. However, the daily administration improved the symptoms related to the depression observed in patients having Parkinsonism.

Therefore, the administration of the compounds according to the invention can improve the quality of life of patients having an alpha-synucleinopathy, especially MSA.

### Example 2- Pilot study of the tolerance and efficacy of taking a dietary supplement based on nicotinamide mononucleotide (NMN) for 3 months in patients suffering from Multiple Systematised Atrophy (MSA): Multicentric, prospective, non-randomised, non-controlled study.

The purpose of the multicentric, prospective, non-randomised, non-controlled pilot study is to study the efficacy of NMN administration in MSA patients (45 - 80 years old) at a larger scale.

In this research, the safety, tolerance and efficacy of a dose of NMN are studied. The amount of NMN to be ingested daily is divided into 2 capsules (Size 0). Each patient takes 2 capsules/day in the morning during breakfast with a big glass of water. The composition of each capsule is as follows:

**Table 7:**

| Ingredient | Role | Weight (mg) |
|---|---|---|
| Beta-NMN (Compound IA) | Active ingredient | 200 |
| Isomalt PE | Excipient | 170 |
| Magnesium stearate | Excipient | 5 |
| Microcrystalline cellulose | Excipient | 20 |

The product is distributed to patients in pill boxes of 60 capsules. Three pillboxes are given to the patient by the neurologist-investigator; the dispensing is done as follows: 1 pill box at inclusion (visit 1), 1 pill box at 15 days (visit 2) and 1 pill box at D45 (visit 3).

The main objectives of the study are to evaluate the safety of the dietary supplement during a 12-week supplementation.

The safety of the food supplement is assessed in terms of:
a) Hemodynamic parameters:
   - Blood pressure (standing up/ lying down (2min)),
   - Heart rate (standing up/ lying down (2min)).
b) Blood Formula Counting:
   - Hematies,
   - Leukocytes (lymphocytes, monocytes, neutrophils, basophils, eosinophils),
   - Platelets.
c) Inflammatory parameters:
   - C Reactive Protein (CRP),
   - Sedimentation rate.
d) Liver function:
   - ASAT,
   - ALAT,
   - gamma GT,
   - Alkaline phosphatases.
e) Kidney function:
   - Creatinemia,
   - Urea.
      Cardiac and muscular function:
   - Creatine Phosphokinase (CPK).
f) Metabolic / biochemical parameters:
   - Ionogram (Na, K, CI),
   - glycated hemoglobin
g) Occurrence of undesirable effects.

48 hours before each follow-up visit by the neurological investigator, a nurse visits the patient's home to take blood samples and measure the haemodynamic parameters [PAS/PAD lying down, standing up (2min) and FC lying down, standing up (2min)]. In addition, during each visit, the nurse questions the patient on the possible occurrence of adverse events. The nurse visits are scheduled between D-7 and D-2 to confirm the patient's inclusion, then at D13 (for visit V2 at D15), D43 (for visit V3 at D45) and D88 (for the end of study visit at D90). No fasting was required for the biologic assessments.

To ensure the safety of the food supplement, all biological parameters must be within the limits of reference values and/or judged by the investigating physician as non-clinical.

The secondary objectives describe the characteristics of the patients who participated in the research, to study the pharmacokinetics of NMN in daily oral use and its impact on increasing NAD+ levels, and to study, in a preliminary way, the potential benefits of NMN supplementation on the symptomatology related to multi-systemic atrophy syndrome such as activities of daily living, motor disorders, orthostatic hypotension and autonomy using the validated UMSARS scale.

**Table 8 - Summary of the objectives of the study and criteria**

| Objectives | Assessment criteria |
|---|---|
| Tolerance | At each visit (V2 to V4), the investigating doctor evaluates the global tolerance to the product in the light of the patient's questioning, the follow-up notebook, the clinical examination including the hemodynamic parameters and the results of the biological analyses. Tolerance is assessed using a 4-point Likert scale. |
| Efficiency | At visits V1, V3 and V4, the investigating neurologist evaluates the efficacy of the product using the UMSARS scale specifically assessing 4 axes: |
| | I. Activities of daily living (12 items), |
| | II. Motor examination (14 items), |
| | III. Orthostatic hypotension [4 measures: BP standing/sleeping (2 min); HR standing/sleeping (2 min) and orthostatic symptoms Y/N] and to conclude, rule on its level of disability (1 item). |
| | IV. Severity of the pathology |
| | Parts I and II are scored out of 48 and 56 respectively. Part III gives the values for the PAS/PAD and HR standing and lying down after 2 minutes of rest and conclude whether or not orthostatic signs are present. Part IV rates the severity of the pathology (1 not very severe to 5 very severe). The evolution of these scores (change from Baseline (V1)) is studied at times V3 and V4. |
| Pharmacokinetics | Prior to each visit (V1 to V4), a blood sample is taken at the patient's home by the IDE to measure the level of NAD in the blood and other NMN metabolites. |
| Impact on quality of life | At visits V1, V2, V3 and V4, the patient/carer assesses their quality of life using the MSA-QoL specific quality of life self-questionnaire. From this questionnaire a score is calculated. The evolution of the score (change from Baseline (V1)) is studied at times V2, V3 and V4. |
| Compliance with the dietary supplement | Each patient has a daily monitoring notebook enabling him/her to record the daily intake of the food supplement. This diary is checked by the nurse during her visits to the patient's home. Also, at visits V2, V3 and V4, the patient has to bring back his pill box and his diary so that the investigator can count the number of capsules actually taken and give him a new follow-up diary. The level of compliance is judged on the basis of these elements. |

Inclusion criteria: The characteristics required for a patient to take part in research are:
- Male or female aged between 45 and 80 years old,
- Non-smoking,
- Presenting an ASM with a predominantly dopa-resistant, cerebellar or dysautonomic Parkinsonism.
- And whose severity of the pathology is deemed compatible with the patient's participation in the study according to the opinion of the investigator (neurologist treating the patient).
- Treatments taken by the patient should be stable and unmodified 1 month before inclusion and throughout the study period for both AMM and psychotropic treatments.
- Available to attend the visits provided for in the protocol and able to complete the data collection documents (compliance and quality of life scale),
- Having given its informed consent in writing to participate in the protocol.

Non-inclusion criteria are:
- Known or suspected allergy to one of the ingredients of the food supplement,
- Presenting, according to the opinion of the investigator, a clinically significant abnormality with regard to the clinical examination and the haematological and/or biochemical parameters during the biological assessment at inclusion,
- Have had cancer in the last 2 years or currently have cancer,
- Having consumed vitamin or food supplements containing niacin, tryptophan, NMN or NR in the 2 months prior to selection,
- Difficulty swallowing capsules,
- An uncontrolled progressive disease outside the AMM,
- Presenting an inability to provide blood samples (bad venous capital),
- Any other condition that, in the opinion of the Investigator, could impair the Investigator's ability to carry out the study or could pose a significant risk,
- Simultaneously participating in another clinical research protocol or having recently participated in another research for which the exclusion period would not be completed.

In addition, the vulnerable persons referred to in Articles L. 1121-5 to 8 and L. 1122-1-2 of the Public Health Code are excluded from the study:
- Pregnant, breastfeeding or parturient women,
- Persons deprived of liberty by a judicial or administrative decision, hospitalized without consent or admitted to a health or social institution for purposes other than research,
- Minors,
- Major citizens under legal protection or unable to express their consent,
- People in emergency situations who cannot express prior consent.

While participating in the study, the patient should observe the following instructions:
- Prohibition to consume vitamin or food supplements during the whole period of participation in the study, (is it not necessary to specify what to do if they are taking therapeutic vitamin D, (and vitamin C) which should not interfere).
- Do not change your eating habits,
- Do not change your caffeine intake (coffee, tea, energy drinks),
- Do not consume grapefruit (whole fruit and/or juice),
- Do not change the frequency, rhythm, and duration of physical activities.

### Inclusion bioassessment (V1) - between D-7 to D-3:

This blood test includes:
- Blood Formula Counts (NFS),
- Metabolic / biochemical parameters: lonogram (Na, K, CI),
- Inflammatory assessment: CRP, sedimentation rate,
- Hepatic assessment: ASAT, ALAT, gamma GT, PAL,
- Renal assessment: Creatinemia, urea,
- Cardiac and muscular assessment: CPK,

Also, an aliquot of serum is stored at the laboratory at -80°C for the determination of the patient's physiological NAD+ (Baseline) level by the promoter. The results are sent to the investigating responsible for checking these parameters (regarding the laboratory's standards) and validating or not the patient's eligibility. These results are the data corresponding to the Baseline.

### Inclusion Visit - D0 (V1)

Prior to the inclusion visit and depending on the results of the blood parameters, the patient's eligibility is validated or not by the neurologist-investigator.

For each eligible patient: during the first visit, the investigator carries out a specific complementary clinical examination MSA (validated specific scale: UMSARS) in order to score the basal level of the patient at inclusion on 4 axes: activities of daily living (12 items), motor level (14 items), orthostatic hypotension [4 measures: BP standing/sleeping (2 min); CF standing/sleeping (2 min) and orthostatic symptoms [Y/N] and finally, decide on his level of disability (1 item). During this visit, he is given pillbox N°1. He is told to start the product under study the next morning, on D1. In fact, the first intake of the dietary supplement can be taken the next morning with breakfast (2 capsules). Pill box N°1 must be returned at visit V2 (D15) to check compliance. A first daily follow-up notebook (follow-up notebook n°1) is given to the patient to record the intake of the food supplement, the concomitant treatments (if applicable) and any undesirable events that may have occurred. Follow-up notebook n°1 should be returned to the investigator at the V2 follow-up visit. The nurse goes to the patient's home 2 days before each follow-up visit in order to carry out the blood test (safety biology), take the hemodynamic constants standing / lying down (after a 2 minute rest): PAS/PAD and FC which is filled in the patient's follow-up notebook. The nurse checks the patient's compliance with the pillbox and the follow-up booklet. The nurse also reminds the patient not to forget to bring back the pill dispenser and the follow-up booklet at each visit. The investigator also takes the hemodynamic constants of the patient standing and lying down (after 2 minutes rest): PAS / PAD and FC. During this consultation, the patient/carer is also asked to answer a specific quality of life questionnaire (MSA-QoL questionnaire). If the patient meets all the eligibility criteria (apart from biology), the neurologist gives him a medical prescription to carry out a biological assessment. This biological assessment must be carried out within 3 days depending on the patient's availability. To do this, a nurse comes to the patient's home to take the sample and then send it to the Medical Analysis Laboratories, the local reference laboratory for this research.

### For each follow-up visit - D13&15 (Visit 2); D43& 45 (Visit 3); D88 & 90 (Visit 4)

Safety biology, PK and hemodynamic constants (D13; D43; D88): A blood sample is taken by the nurse at the patient's home 48 hours before the visit (D13; D43 and D88) so that the investigator has the biological safety results on the day of the consultation.

The required bioassessment is the same as the inclusion one (D-2) and include:
- Complete and differential Blood cells Counts,
- Metabolic / biochemical parameters: lonogram (Na, K, CI), Glycated haemoglobin (HbA1C)
- Inflammatory assessment: CRP, sedimentation rate,
- Liver check-up: ASAT, ALAT, gamma GT, PAL, bilirubin
- Renal assessment: Creatinemia, urea,
- Cardiac and muscular assessment: CPK;

Also, an aliquot of acidified whole blood is stored at the AML at -80°C for the determination of NMN metabolites. The hemodynamic constants (BP and HR standing up/lying down) are taken and entered in the patient's follow-up notebook by the nurse. The nurse also questions the patient on the possible occurrence of adverse events and checks the patient's compliance with treatment by filling in his or her follow-up notebook and pill box. On receipt, the investigating physician check the results: the blood parameters must be within the reference value limits and/or judged by the investigating physician as not clinically relevant. Otherwise, the patient is summoned without delay by the investigating physician to leave the study prematurely.

### Follow-up visits at D15; D45 and D90: Investigator-neurologist

At this visit, the investigator checks the patient's completed follow-up notebook (compliance, adverse event (AE), treatments) and carries out an interrogation and clinical examination, including the taking of standing and lying hemodynamic constants (PAS/PAD and FC) in order to check the tolerance and the possible occurrence of an adverse event. Compliance is also monitored. The patient is asked to complete a quality of life questionnaire (MSA-QoL).

During the third visit at D45 and fourth visit at D90, the investigator also carries out a specific complementary clinical examination MSA (validated specific scale: UMSARS) in order to score the patient's level after 3 months of NMN supplementation on 4 axes : activities of daily living (12 items), motor level (14 items), orthostatic hypotension [4 measures: BP standing/sleeping (2 min); HR standing/sleeping (2 min) and orthostatic symptoms Y/N] and finally decide on his level of disability (1 item).

At D90, the safety and tolerance of the study product is assessed by the investigating physician during the visits provided for in the protocol by means of an interview with the patient, information gathered by the volunteer in the daily diary, a clinical examination (including blood pressure and heart rate measurements) and a blood test.
*

The inventors have thus demonstrated that the use of compounds of formula (I) and formula (Ia) reduces the symptoms of alpha-synucleinopathies such as DLB and MSA, preferably MSA.

### Example 3 - Synthesis of compound of formula I wherein Synthesis of compounds of formula I wherein

R7 is 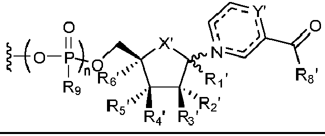 and n is 1:
As disclosed herein, a compound of formula I wherein R7 is 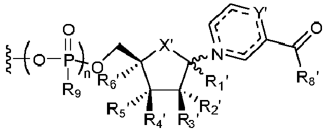 and n is 1, the synthesis can involve in a first step the mono-phosphorylation of alpha-nicotinamide riboside, in the presence of phosphoryl chloride and a trialkyl phosphate, to yield the phosphorodichloridate:

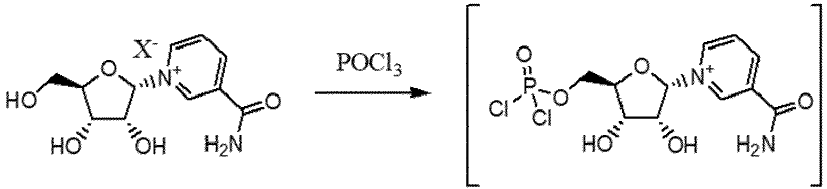

Compound I-B (alpha NMN) is then added as follows:

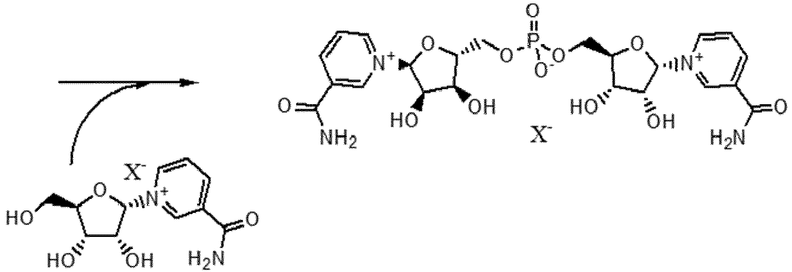

Alternatively, the compound according to the invention can be prepared by the activation of compound I-B by the addition of carbonyldiimidazole (CDI): 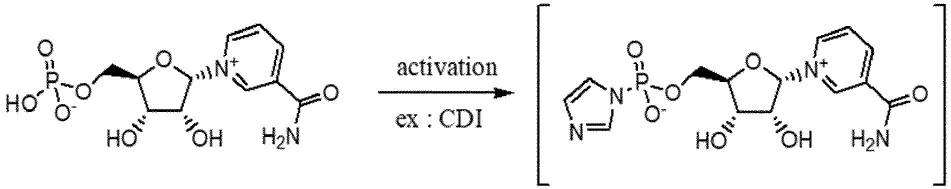 to which alpha -nicotinamide riboside is added as follows:

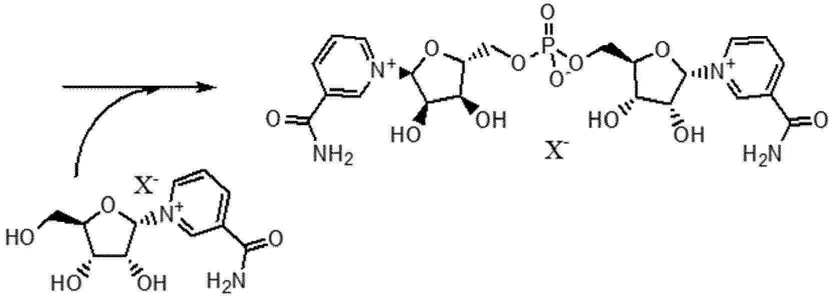

### Example 4 - Synthesis of compound of formula I wherein Synthesis of compounds of formula I wherein R7 is

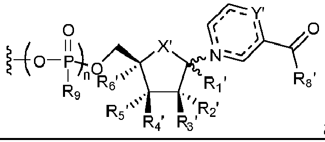

and n is 3:
A compound of formula I comprising three phosphate groups can be prepared as follows. Compound I-B can be activated by the addition of carbonyldiimidazole (CDI):

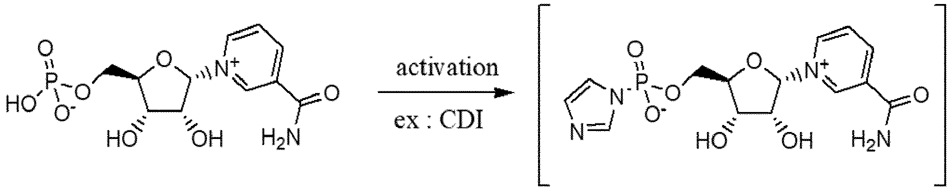

To which a tertbutyleamine -phosphate is added:

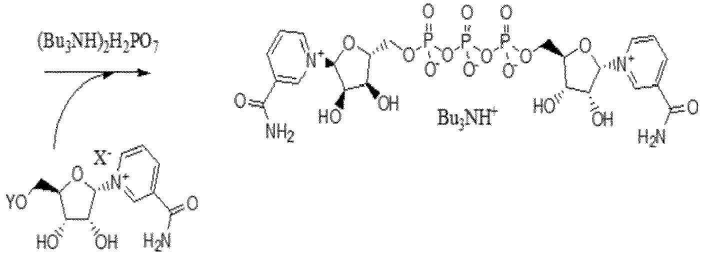

## Claims

1. Compound of formula (I): 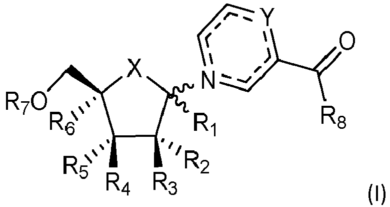 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 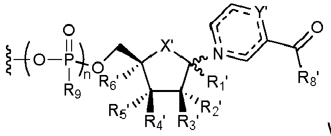 wherein n is an integer chosen amongst 1 or 3; wherein:
R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
R11 is selected from C1-C10 alkyl, C3-C10 cycloalkyl, C5-C18 aryl, C1-C10 alkylaryl, substituted C5-C12 aryl, C1-C10 heteroalkyl, C3-C10 heterocycloalkyle, C1-C10 haloalkyl, heteroaryl, -(CH2)nC(O)(C1-C15)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of R11a is independently selected from H and (C1-C6) alkyl and R11b is independently selected from OH, C1-C6 alkoxy, NH2, NH(C1-C6 alkyl) or N(C1-C6 alkyl)2 ;
R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
R13 and R14 are independently selected from H, C1-C8 alkyl and C1-C8 alkyl aryl;
Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
R9 and R10 together with the phosphorus atoms to which they are attached form a 6-membered ring wherein -R9-R10- represents -O-CH2-CH2-CHR-O-; wherein R is selected from hydrogen, C5-C6 aryl and
C5-C6 heteroaryl, wherein said aryl or heteroaryl groups are optionally substituted by one or two groups selected from halogen, trifluoromethyl, C1-C6 alkyl, C1-C6 alkoxy and cyano;
R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or
more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or
more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl;
Y is selected from CH, CH2, C(CH3)2 and CCH3;
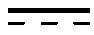 represents a single or double bond according to Y; and
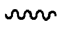 represents the alpha or beta anomer depending on the position of R1,
or a compound of formula (Ia) 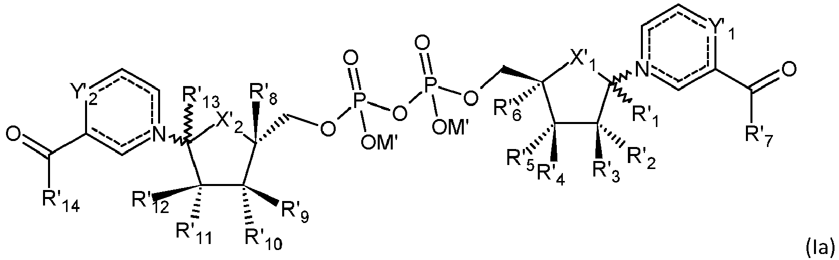 or pharmaceutically acceptable salts, hydrates, crystals, stereoisomers and/or solvates thereof, wherein:
X'1 and X'2 are independently selected from O, CH2, S, Se, CHF, CF2 and C=CH2;
R'1 and R'13 are independently selected from H, azido, cyano, C1-C8 alkyl, C1-C8 thio-alkyl, C1-C8 heteroalkyl and OR; wherein R is selected from H and C1-C8 alkyl;
R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid ;
R'6 and R'8 are independently selected from H, azido, cyano, C1-C8 alkyl and OR; wherein R is selected from H and C1-C8 alkyl;
R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
Y'1 and Y'2 are independently selected from CH, CH2, C(CH3)2 or CCH3;
M' is selected from H or a suitable counter-ion;
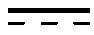 represents a single or a double bound depending on Y'1 and Y'2; and
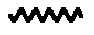 represents the alpha or beta anomer depending on the position of R'1 and R'13,
and their combinations for its use in the prevention and/or treatment of an alpha-synucleinopathy chosen amongst dementia with Lewy bodies (DLB) and multiple system atrophy (MSA).

2. Compound of formula (I) and/or compound of formula (Ia) for their use according to claim 1 wherein the alpha-synucleinopathy is MSA.

3. Compound of formula (I) and/or compound of formula (Ia) for their use according to claim 1 wherein the alpha-synucleinopathy is not Parkinson disease.

4. Compound of formula (I) and/or compound of formula (Ia) for their use according to claim 2 wherein the MSA is chosen amongst MSA-predominant Parkinsonism (MSA-P) or the MSA-predominant cerebellar ataxia (MSA-C).

5. Compound of formula (I) and/or compound of formula (Ia) for their use according to any of the preceding claims wherein compound of formula (I) is chosen amongst dihydro-nicotinamide mononucleotide, compounds I-A, compound I-B, compound I-C, compound I-D, compound I-E, compound I-F, compound I-G, compound I-H, compound I-I, compound I-J as shown in table 1, preferably I-B, I-D or I-F.

6. Compound of formula (I) and/or compound of formula (Ia) for their use according to any of the preceding claims wherein compound of formula (Ia) is chosen amongst compounds la-A, compound la-B, compound la-C, compound la-D, compound la-E, compound la-F, compound la-G, compound la-H, compound Ia-I as shown in table 2.

7. Compound of formula (I) and/or compound of formula (Ia) for their use according to any of the preceding claims in combination with at least one further therapeutic ingredient.

8. Compound of formula (I) and/or compound of formula (Ia) for their use according to claim 7 wherein the at least one further therapeutic ingredient is chosen amongst a dopaminergic precursor, a monoamine oxidase type B inhibitor, an anticholinergic agent, a dopaminergic agonist, a noradrenaline precursor, a muscarinic antagonist, an alpha-blocker, a beta-adrenergic agonist, a corticoid, a phosphodiesterase inhibitor, a vasopressin V2 receptor agonist and their combinations.

9. Composition comprising at least one compound of formula (I): 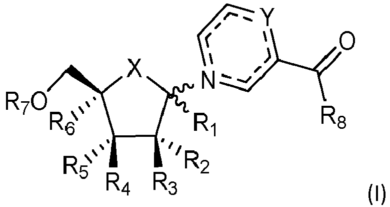 or pharmaceutically acceptable salts, hydrates, crystals, stereoisomers and/or solvates thereof, wherein:
X is selected from O, CH2, S, Se, CHF, CF2 et C=CH2;
R1 is selected from H, azido, cyano, C1-C8 alkyl, C1-C8 thio-alkyl, C1-C8 heteroalkyl and OR; wherein R is selected from H and C1 C8 alkyl;
R2, R3, R4 et R5 are independently selected from H, halogen, azido, cyano, hydroxyl, C1-C12 alkyl, C1-C12 thioalkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl and C(O)CHRAANH2 ; wherein RAA is a side chain selected from a proteinogenic amino acid;
R6 is selected from H, azido, cyano, C1-C8 alkyl, C1-C8 thio-alkyl, C1-C8 heteroalkyl and OR; wherein R is selected from H and C1 C8 alkyl;
R7 is selected from P(O)R9R10, P(S)R9R10 and 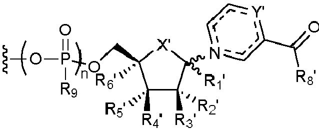 wherein n is an integer chosen amongst 1 or 3; wherein:
R9 and R10 are independently selected from O-, OH, OR11, NHR13, NR13R14, C1-C8 alkyl, C2-C8 alkenyl, C2-C8 alkynyl, C3-C10 cycloalkyl, C5-C12 aryl, C1-C8 arylalkyl, C1-C8 alkylaryl, C1-C8 heteroalkyl, C1-C8 heterocycloalkyl, heteroaryl and NHCRαRα'C(O)R12; wherein:
R11 is selected from C1-C10 alkyl, C3-C10 cycloalkyl, C5-C18 aryl, C1-C10 alkylaryl, substituted C5-C12 aryl, C1-C10 heteroalkyl, C3-C10 heterocycloalkyle, C1-C10 haloalkyl, heteroaryl, -(CH2)nC(O)(C1-C15)alkyl, - (CH2)nOC(O)(C1-C15)alkyl, -(CH2)nOC(O)O(C1-C15)alkyl, -(CH2)nSC(O)(C1-C15)alkyl, -(CH2)nC(O)O(C1-C15)alkyl and -(CH2)nC(O)O(C1-C15)alkyl aryl; wherein n is an integer selected from 1 to 8; P(O)(OH)OP(O)(OH)2; halogen, nitro, cyano, C1-C6 alkoxy, C1-C6 haloalkoxy, -N(R11a)2, C1-C6 acylamino, -COR11b, -O COR11b; NHSO2(C1-C6 alkyl), - SO2N(R11a)2 SO2 wherein each of R11a is independently selected from H and (C1-C6) alkyl and R11b is independently selected from OH, C1-C6 alkoxy, NH2, NH(C1-C6 alkyl) or N(C1-C6 alkyl)2;
R12 is selected from hydrogen, C1-C10 alkyl, C2-C8 alkenyl, C2-C8 alkynyl, C1 C10 haloalkyl, C3-C10 cycloalkyl, C3-C10 cycloheteroalkyl, C5-C12 aryl, C1-C4 alkylaryl and C5-C12 heteroaryl, wherein said aryl or heteroaryl groups are optionally substituted by one or two groups selected from halogen, trifluoromethyl, C1-C6 alkyl, C1-C6 alkoxy and cyano;
R13 and R14 are independently selected from H, C1-C8 alkyl and C1-C8 alkyl aryl;
Rα and Rα' are independently selected from an hydrogen, C1-C10 alkyl, C2-C10 alkenyl, C2-C10 alkynyl, C3-C10 cycloalkyl, C1-C10 thio-alkyl, C1-C10 hydroxylalkyl, C1-C10 alkylaryl and C5-C12 aryl, - (CH2)3NHC(=NH)NH2, (1H-indol-3-yl)methyl, (1H-imidazol-4-yl)methyl and a side chain selected from a proteinogenic or non-proteinogenic amino acid; wherein said aryl groups are optionally substituted with a group selected from hydroxyl, C1-C10 alkyl, C1-C6 alkoxy, halogen, nitro and cyano; or
R9 and R10 together with the phosphorus atoms to which they are attached form a 6-membered ring wherein -R9-R10- represents -CH2-CH2-CHR-; wherein R is selected from hydrogen, C5-C6 aryl and C5-C6 heteroaryl; wherein said aryl or heteroaryl groups are optionally substituted by one or two groups selected from halogen, trifluoromethyl, C1-C6 alkyl, C1-C6 alkoxy and cyano; or
R9 and R10 together with the phosphorus atoms to which they are attached form a 6-membered ring wherein -R9-R10- represents -O-CH2-CH2-CHR-O-; wherein R is selected from hydrogen, C5-C6 aryl and C5-C6 heteroaryl, wherein said aryl or heteroaryl groups are optionally substituted by one or two groups selected from halogen, trifluoromethyl, C1-C6 alkyl, C1-C6 alkoxy and cyano;
R8 is selected from H, OR, NHR15, NR15R16, NH-NHR13, SH, CN, N3 and halogen; wherein R15 and R16 are independently selected from H, C1-C8 alkyl and C1-C8 alkyl aryl; and -CRBRC-C(O)-ORD wherein RB and RC are independently hydrogen, C1-C6 alkyl, C1-C6 alkoxy, benzyl, indolyl or imidazolyl, wherein the C1-C6 alkyl and C1-C6 alkoxy may be optionally and independently of each other substituted by one or more of halogen, amino, amido, guanidyl, hydroxyl, thiol or carboxyl groups, and the benzyl group is optionally substituted by one or more of the halogen or hydroxyl groups, or RB and RC together with the carbon atom to which they are attached form a C3-C6 cycloalkyl group optionally substituted by one or more halogen, amino, amido, guanidyl, hydroxyl, thiol and carboxyl groups and RD is hydrogen, C1-C6 alkyl, C2-C6 alkenyl, C2-C6 alkynyl or C3-C6 cycloalkyl;
Y is selected from CH, CH2, C(CH3)2 and CCH3;
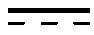 represents a single or double bond according to Y; and
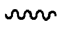 represents the alpha or beta anomer depending on the position of R1,
or a compound of formula (Ia) 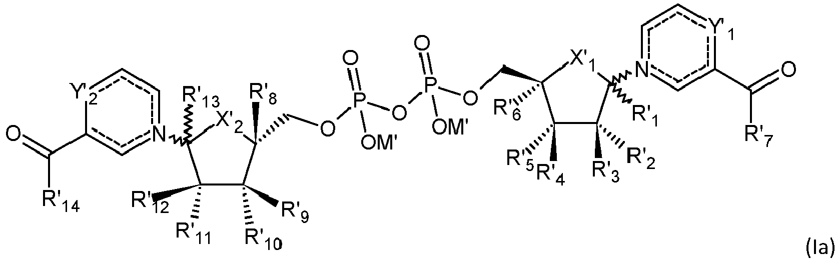 or pharmaceutically acceptable salts, hydrates, crystals, stereoisomers and/or solvates thereof, wherein:
X'1 and X'2 are independently selected from O, CH2, S, Se, CHF, CF2 and C=CH2;
R'1 and R'13 are independently selected from H, azido, cyano, C1-C8 alkyl, C1-C8 thio-alkyl, C1-C8 heteroalkyl and OR; wherein R is selected from H and C1-C8 alkyl;
R'2, R'3, R'4, R'5, R'9, R'10, R'11, R'12 are independently selected from H, halogen, azido, cyano, hydroxyl, C1-C12 alkyl, C1-C12 thio-alkyl, C1-C12 heteroalkyl, C1-C12 haloalkyl and OR; wherein R is selected from H, C1-C12 alkyl, C(O)(C1-C12)alkyl, C(O)NH(C1-C12)alkyl, C(O)O(C1-C12)alkyl, C(O)aryl, C(O)(C1-C12)alkyl aryl, C(O)NH(C1-C12)alkyl aryl, C(O)O(C1-C12)alkyl aryl or C(O)CHRAANH2, wherein RAA is a side chain selected from a proteinogenic amino acid ;
R'6 and R'8 are independently selected from H, azido, cyano, C1-C8 alkyl and OR; wherein R is selected from H and C1-C8 alkyl;
R'7 and R'14 are independently selected from H, OR, NHR, NRR', NH-NHR, SH, CN, N3 and halogen; wherein R and R' are each independently selected from H, C1-C8 alkyl, C1-C8 alkyl aryl;
Y'1 and Y'2 are independently selected from CH, CH2, C(CH3)2 or CCH3;
M' is selected from H or a suitable counter-ion;
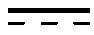 represents a single or a double bound depending on Y'1 and Y'2; and
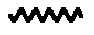 represents the alpha or beta anomer depending on the position of R'1 and R'13,
and their combinations, for use in the prevention and/or of a alpha-synucleinopathy.

10. Composition for use according to claim 9 wherein the alpha- synucleinopathy is chosen amongst dementia with Lewy bodies (DLB) and multiple system atrophy (MSA), preferably MSA.

11. Composition for use according to any of claims 9-10 further comprising at least one further therapeutic ingredient.

12. Composition for use according to claim 11 wherein the at least one further therapeutic ingredient is chosen amongst chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations.

13. Kit of parts comprising at least one compound of formula (I) and/or one compound of formula (Ia) according to any of claims 1-8 or one composition according to any of claims 9-12, and at least one further part.

14. Kit of part according to claim 13 wherein the at least one further part is a further therapeutic ingredient, preferably chosen amongst levodopa, carbidopa, droxidopa, oxybutine chloride, tolterodine, tamsulosine, mirabegron, tadalafil, sildenafil, midodrine, fludrocortisone, desmopressine, a laxative, an anticholinergic agent, an acetylcholinesterase (ACE) inhibitor, a benzodiazepine, a dopaminergic agent, zolpidem, modafinil, armodafinil and their combinations.
